# EUROPEAN PATENT SPECIFICATION

(11) **EP 3 009 143 B1**
(45) Date of publication and mention of the grant of the patent: **03.10.2018**
(21) Application number: 15194111.9
(22) Date of filing: 16.01.2009
(51) Int. Cl.: A61K 38/17, A61K 45/00, A61P 25/16, A61P 25/28, A61K 31/70

(54) **PROGRANULIN FOR USE IN TREATING PARKINSON'S DISEASE OR ALZHEIMER'S DISEASE**
PROGRANULIN ZUR BEHANDLUNG DER PARKINSON'SCHEN KRANKHEIT ODER ALZHEIMER-KRANKHEIT
PROGRANULINE POUR UNE UTILISATION DANS LE TRAITEMENT DE LA MALADIE DE PARKINSON OU LA MALADIE D'ALZHEIMER

(30) Priority: 16.01.2008 US 11253; 16.01.2008 US 11284
(43) Date of publication of application: 20.04.2016
(62) Divisional of application: 09701647.1
(73) Proprietor: Neurodyn Life Sciences Inc., Charlottetown, Prince Edward Island., C1A 4P3 (CA)
(72) Inventor: KAY, Denis G., Stratford PE C1B 1Y 8 (CA)
(74) Representative: Grünecker Patent- und Rechtsanwälte PartG mbB

(56) References cited:
- WO-A1-2009/010045
- WO-A2-2007/146046
- WO-A2-2008/019187
- ZHIHENG HE ET AL: "Progranulin is a mediator of the wound response", NATURE MEDICINE, vol. 9, no. 2, 1 February 2003 (2003-02-01), pages 225-229, XP055032993, ISSN: 1078-8956, DOI: 10.1038/nm816
- SIEGERS K ET AL: "Progranulin modifies onset age and survival in Amyotrophic Lateral Sclerosis", NEUROLOGY, LIPPINCOTT WILLIAMS & WILKINS, PHILADELPHIA, US, vol. 68, no. 12, SU, 20 March 2007 (2007-03-20), page A202, XP008140330, ISSN: 0028-3878
- PHILIP VAN DAMME ET AL: "Progranulin functions as a neurotrophic factor to regulate neurite outgrowth and enhance neuronal survival", THE JOURNAL OF CELL BIOLOGY : JCB, THE ROCKEFELLER UNIVERSITY PRESS, US, vol. 181, no. 1, 7 April 2008 (2008-04-07) , pages 37-41, XP008140331, ISSN: 0021-9525, DOI: 10.1083/JCB.200712039
- CRUTS M ET AL: "Progranulin mutations in Ubiquitin-positive frontotemporal dementia linked to Chromosome 17q21", CURRENT ALZHEIMER RESEARCH, BENTHAM SCIENCE PUBL. LTD, NL, vol. 3, no. 5, 1 January 2006 (2006-01-01) , pages 485-491, XP008140332, ISSN: 1567-2050
- YONG-JIE ZHANG ET AL: "Progranulin mediates caspase-dependent cleavage of TAR DNA Binding Protein-43", JOURNAL OF NEUROSCIENCE, THE SOCIETY, WASHINGTON, DC, US, vol. 27, no. 39, 26 September 2007 (2007-09-26), pages 10530-10534, XP008140333, ISSN: 1529-2401, DOI: 10.1523/JNEUROSCI.3421-07.2007

### SEQUENCE LISTING

<110> NEURODYN, INC.
<120> TREATING NEURODEGENERATIVE DISEASES WITH PROGRANULIN (PGRN)
<130> EP71914IHV268pau
<140> not yet assigned
   <141> 2009-01-16
<150> 09 701 647.1
   <151> 2009-01-16
<150> PCT/CA2009/000074
   <151> 2009-01-16
<150> 61/011,253
   <151> 2008-01-16
<150> 61/011,284
   <151> 2008-01-16
<160> 19
<170> PatentIn version 3.5
<210> 1
   <211> 2323
   <212> DNA
   <213> Homo sapiens
<400> 1
   ggcgagagga agcagggagg agagtgattt gagtagaaaa gaaacacagc attccaggct       60
   ggccccacct ctatattgat aagtagccaa tgggagcggg tagccctgat ccctggccaa      120
   tggaaactga ggtaggcggg tcatcgcgct ggggtctgta gtctgagcgc tacccggttg      180
   ctgctgccca aggaccgcgg agtcggacgc aggcagacca tgtggaccct ggtgagctgg      240
   gtggccttaa cagcagggct ggtggctgga acgcggtgcc cagatggtca gttctgccct      300
   gtggcctgct gcctggaccc cggaggagcc agctacagct gctgccgtcc ccttctggac      360
   aaatggccca caacactgag caggcatctg ggtggcccct gccaggttga tgcccactgc      420
   tctgccggcc actcctgcat ctttaccgtc tcagggactt ccagttgctg ccccttccca      480
   gaggccgtgg catgcgggga tggccatcac tgctgcccac ggggcttcca ctgcagtgca      540
   gacgggcgat cctgcttcca aagatcaggt aacaactccg tgggtgccat ccagtgccct      600
   gatagtcagt tcgaatgccc ggacttctcc acgtgctgtg ttatggtcga tggctcctgg      660
   gggtgctgcc ccatgcccca ggcttcctgc tgtgaagaca gggtgcactg ctgtccgcac      720
   ggtgccttct gcgacctggt tcacacccgc tgcatcacac ccacgggcac ccaccccctg      780
   gcaaagaagc tccctgccca gaggactaac agggcagtgg ccttgtccag ctcggtcatg      840
   tgtccggacg cacggtcccg gtgccctgat ggttctacct gctgtgagct gcccagtggg      900
   aagtatggct gctgcccaat gcccaacgcc acctgctgct ccgatcacct gcactgctgc 960
   ccccaagaca ctgtgtgtga cctgatccag agtaagtgcc tctccaagga gaacgctacc 1020
   acggacctcc tcactaagct gcctgcgcac acagtggggg atgtgaaatg tgacatggag 1080
   gtgagctgcc cagatggcta tacctgctgc cgtctacagt cgggggcctg gggctgctgc 1140
   ccttttaccc aggctgtgtg ctgtgaggac cacatacact gctgtcccgc ggggtttacg 1200
   tgtgacacgc agaagggtac ctgtgaacag gggccccacc aggtgccctg gatggagaag 1260
   gccccagctc acctcagcct gccagaccca caagccttga agagagatgt cccctgtgat 1320
   aatgtcagca gctgtccctc ctccgatacc tgctgccaac tcacgtctgg ggagtggggc 1380
   tgctgtccaa tcccagaggc tgtctgctgc tcggaccacc agcactgctg cccccagggc 1440
   tacacgtgtg tagctgaggg gcagtgtcag cgaggaagcg agatcgtggc tggactggag 1500
   aagatgcctg cccgccgggc ttccttatcc caccccagag acatcggctg tgaccagcac 1560
   accagctgcc cggtggggca gacctgctgc ccgagcctgg gtgggagctg ggcctgctgc 1620
   cagttgcccc atgctgtgtg ctgcgaggat cgccagcact gctgcccggc tggctacacc 1680
   tgcaacgtga aggctcgatc ctgcgagaag gaagtggtct ctgcccagcc tgccaccttc 1740
   ctggcccgta gccctcacgt gggtgtgaag gacgtggagt gtggggaagg acacttctgc 1800
   catgataacc agacctgctg ccgagacaac cgacagggct gggcctgctg tccctaccgc 1860
   cagggcgtct gttgtgctga tcggcgccac tgctgtcctg ctggcttccg ctgcgcagcc 1920
   aggggtacca agtgtttgcg cagggaggcc ccgcgctggg acgccccttt gagggaccca 1980
   gccttgagac agctgctgtg agggacagta ctgaagactc tgcagccctc gggaccccac 2040
   tcggagggtg ccctctgctc aggcctccct agcacctccc cctaaccaaa ttctccctgg 2100
   accccattct gagctcccca tcaccatggg aggtggggcc tcaatctaag gccttccctg 2160
   tcagaagggg gttgtggcaa aagccacatt acaagctgcc atcccctccc cgtttcagtg 2220
   gaccctgtgg ccaggtgctt ttccctatcc acaggggtgt ttgtgtgtgt gcgcgtgtgc 2280
   gtttcaataa agtttgtaca ctttcaaaaa aaaaaaaaaa aaa 2323
<210> 2
   <211> 593
   <212> PRT
   <213> Homo sapiens
<400> 2
<210> 3
   <211> 57
   <212> PRT
   <213> Homo sapiens
<400> 3
   Asp Val Lys Cys Asp Met Glu Val Ser Cys Pro Asp Gly Tyr Thr Cys
   1 5 10 15
   Cys Arg Leu Gln Ser Gly Ala Trp Gly Cys Cys Pro Phe Thr Gln Ala
   20 25 30
   Val Cys Cys Glu Asp His Ile His Cys Cys Pro Ala Gly Phe Thr Cys
   35 40 45
   Asp Thr Gln Lys Gly Thr Cys Glu Gln
   50 55
<210> 4
   <211> 57
   <212> PRT
   <213> Homo sapiens
<400> 4
   Val Met Cys Pro Asp Ala Arg Ser Arg Cys Pro Asp Gly Ser Thr Cys
   1 5 10 15
   Cys Glu Leu Pro Ser Gly Lys Tyr Gly Cys Cys Pro Met Pro Asn Ala
   20 25 30
   Thr Cys Cys Ser Asp His Leu His Cys Cys Pro Gln Asp Thr Val Cys
   35 40 45
   Asp Leu Ile Gln Ser Lys Cys Leu Ser
   50 55
<210> 5
   <211> 54
   <212> PRT
   <213> Homo sapiens
<400> 5
   Val Pro Cys Asp Asn Val Ser Ser Cys Pro Ser Ser Asp Thr Cys Cys
   1               5                   10                  15
   Gln Leu Thr Ser Gly Glu Trp Gly Cys Cys Pro Ile Pro Glu Ala Val
               20                  25                  30
   Cys Cys Ser Asp His Gln His Cys Cys Pro Gln Gly Tyr Thr Cys Val
           35                  40                  45
   Ala Glu Gly Gln Cys Gln
       50
<210> 6
   <211> 56
   <212> PRT
   <213> Homo sapiens
<400> 6
   Asp Ile Gly Cys Asp Gln His Thr Ser Cys Pro Val Gly Gln Thr Cys
   1               5                   10                  15
   Cys Pro Ser Leu Gly Gly Ser Trp Ala Cys Cys Gln Leu Pro His Ala
               20                  25                  30
   Val Cys Cys Glu Asp Arg Gln His Cys Cys Pro Ala Gly Tyr Thr Cys
           35                  40                  45
   Asn Val Lys Ala Arg Ser Cys Glu
       50                  55
<210> 7
   <211> 56
   <212> PRT
   <213> Homo sapiens
<400> 7
   Asp Val Glu Cys Gly Glu Gly His Phe Cys His Asp Asn Gln Thr Cys
   1               5                   10                  15
   Cys Arg Asp Asn Arg Gln Gly Trp Ala Cys Cys Pro Tyr Arg Gln Gly
               20                  25                  30
   Val Cys Cys Ala Asp Arg Arg His Cys Cys Pro Ala Gly Phe Arg Cys
           35                  40                  45
   Ala Ala Arg Gly Thr Lys Cys Leu
                       50                  55
<210> 8
   <211> 57
   <212> PRT
   <213> Homo sapiens
<400> 8
   Ala Ile Gln Cys Pro Asp Ser Gln Phe Glu Cys Pro Asp Phe Ser Thr
   1               5                   10                  15
   Cys Cys Val Met Val Asp Gly Ser Trp Gly Cys Cys Pro Met Pro Gln
               20                  25                  30
   Ala Ser Cys Cys Glu Asp Arg Val His Cys Cys Pro His Gly Ala Phe
           35                  40                  45
   Cys Asp Leu Val His Thr Arg Cys Ile
       50                  55
<210> 9
   <211> 56
   <212> PRT
   <213> Homo sapiens
<400> 9
   Gly Gly Pro Cys Gln Val Asp Ala His Cys Ser Ala Gly His Ser Cys
   1               5                   10                  15
   Ile Phe Thr Val Ser Gly Thr Ser Ser Cys Cys Pro Phe Pro Glu Ala
               20                  25                  30
   Val Ala Cys Gly Asp Gly His His Cys Cys Pro Arg Gly Phe His Cys
           35                  40                  45
   Ser Ala Asp Gly Arg Ser Cys Phe
       50                  55
<210> 10
   <211> 71
   <212> PRT
   <213> Artificial Sequence
<220>
   <223> Description of Artificial Sequence: Synthetic peptide
<400> 10
   Ala Met Asp Ile Gly Cys Asp Gln His Thr Ser Cys Pro Val Gly Gln
   1               5                   10                  15
   Thr Cys Cys Pro Ser Leu Gly Gly Ser Trp Ala Cys Cys Gln Leu Pro
               20                  25                  30
   His Ala Val Cys Cys Glu Asp Arg Gln His Cys Cys Pro Ala Gly Tyr
           35                  40                  45
   Thr Cys Asn Val Lys Ala Arg Ser Cys Glu Lys Leu Ala Ala Ala Leu
       50                  55                  60
   Glu His His His His His His
   65                  70
<210> 11
   <211> 72
   <212> PRT
   <213> Artificial Sequence
<220>
   <223> Description of Artificial Sequence: Synthetic peptide
<400> 11
   Ala Met Ala Ile Gln Cys Pro Asp Ser Gln Phe Glu Cys Pro Asp Phe
   1               5                   10                  15
   Ser Thr Cys Cys Val Met Val Asp Gly Ser Trp Gly Cys Cys Pro Met
               20                  25                  30
   Pro Gln Ala Ser Cys Cys Glu Asp Arg Val His Cys Cys Pro His Gly
           35                  40                  45
   Ala Phe Cys Asp Leu Val His Thr Arg Cys Ile Lys Leu Ala Ala Ala
       50                  55                  60
   Leu Glu His His His His His His
   65                  70
<210> 12
   <211> 589
   <212> PRT
   <213> Mus sp.
<400> 12
   Met Trp Val Leu Met Ser Trp Leu Ala Phe Ala Ala Gly Leu Val Ala
   1               5                   10                  15
   Gly Thr Gln Cys Pro Asp Gly Gln Phe Cys Pro Val Ala Cys Cys Leu
               20                  25                  30
   Asp Gln Gly Gly Ala Asn Tyr Ser Cys Cys Asn Pro Leu Leu Asp Thr
           35                  40                  45
   Trp Pro Arg Ile Thr Ser His His Leu Asp Gly Ser Cys Gln Thr His
   50 55 60
   Gly His Cys Pro Ala Gly Tyr Ser Cys Leu Leu Thr Val Ser Gly Thr
   65 70 75 80
   Ser Ser Cys Cys Pro Phe Ser Lys Gly Val Ser Cys Gly Asp Gly Tyr
   85 90 95
   His Cys Cys Pro Gln Gly Phe His Cys Ser Ala Asp Gly Lys Ser Cys
   100 105 110
   Phe Gln Met Ser Asp Asn Pro Leu Gly Ala Val Gln Cys Pro Gly Ser
   115 120 125
   Gln Phe Glu Cys Pro Asp Ser Ala Thr Cys Cys Ile Met Val Asp Gly
   130 135 140
   Ser Trp Gly Cys Cys Pro Met Pro Gln Ala Ser Cys Cys Glu Asp Arg
   145 150 155 160
   Val His Cys Cys Pro His Gly Ala Ser Cys Asp Leu Val His Thr Arg
   165 170 175
   Cys Val Ser Pro Thr Gly Thr His Thr Leu Leu Lys Lys Phe Pro Ala
   180 185 190
   Gln Lys Thr Asn Arg Ala Val Ser Leu Pro Phe Ser Val Val Cys Pro
   195 200 205
   Asp Ala Lys Thr Gln Cys Pro Asp Asp Ser Thr Cys Cys Glu Leu Pro
   210 215 220
   Thr Gly Lys Tyr Gly Cys Cys Pro Met Pro Asn Ala Ile Cys Cys Ser
   225 230 235 240
   Asp His Leu His Cys Cys Pro Gln Asp Thr Val Cys Asp Leu Ile Gln
   245 250 255
   Ser Lys Cys Leu Ser Lys Asn Tyr Thr Thr Asp Leu Leu Thr Lys Leu
   260 265 270
   Pro Gly Tyr Pro Val Lys Glu Val Lys Cys Asp Met Glu Val Ser Cys
   275 280 285
   Pro Glu Gly Tyr Thr Cys Cys Arg Leu Asn Thr Gly Ala Trp Gly Cys
   290 295 300
   Cys Pro Phe Ala Lys Ala Val Cys Cys Glu Asp His Ile His Cys Cys
   305                 310                 315                 320
   Pro Ala Gly Phe Gln Cys His Thr Glu Lys Gly Thr Cys Glu Met Gly
                   325                 330                 335
   Ile Leu Gln Val Pro Trp Met Lys Lys Val Ile Ala Pro Leu Arg Leu
               340                 345                 350
   Pro Asp Pro Gln Ile Leu Lys Ser Asp Thr Pro Cys Asp Asp Phe Thr
           355                 360                 365
   Arg Cys Pro Thr Asn Asn Thr Cys Cys Lys Leu Asn Ser Gly Asp Trp
       370                 375                 380
   Gly Cys Cys Pro Ile Pro Glu Ala Val Cys Cys Ser Asp Asn Gln His
   385                 390                 395                 400
   Cys Cys Pro Gln Gly Phe Thr Cys Leu Ala Gln Gly Tyr Cys Gln Lys
                   405                 410                 415
   Gly Asp Thr Met Val Ala Gly Leu Glu Lys Ile Pro Ala Arg Gln Thr
               420                 425                 430
   Thr Pro Leu Gln Ile Gly Asp Ile Gly Cys Asp Gln His Thr Ser Cys
           435                 440                 445
   Pro Val Gly Gln Thr Cys Cys Pro Ser Leu Lys Gly Ser Trp Ala Cys
       450                 455                 460
   Cys Gln Leu Pro His Ala Val Cys Cys Glu Asp Arg Gln His Cys Cys
   465                 470                 475                 480
   Pro Ala Gly Tyr Thr Cys Asn Val Lys Ala Arg Thr Cys Glu Lys Asp
                   485                 490                 495
   Val Asp Phe Ile Gln Pro Pro Val Leu Leu Thr Leu Gly Pro Lys Val
               500                 505                 510
   Gly Asn Val Glu Cys Gly Glu Gly His Phe Cys His Asp Asn Gln Thr
           515                 520                 525
   Cys Cys Lys Asp Ser Ala Gly Val Trp Ala Cys Cys Pro Tyr Leu Lys
       530                 535                 540
   Gly Val Cys Cys Arg Asp Gly Arg His Cys Cys Pro Gly Gly Phe His
   545                 550                 555                 560
   Cys Ser Ala Arg Gly Thr Lys Cys Leu Arg Lys Lys Ile Pro Arg Trp
   565 570 575
   Asp Met Phe Leu Arg Asp Pro Val Pro Arg Pro Leu Leu
   580 585
<210> 13
   <211> 2145
   <212> DNA
   <213> Mus sp.
<400> 13
   gagatgcctc ccagggagcc cggaccccga cgcaggcaga ccatgtgggt cctgatgagc      60
   tggctggcct tcgcggcagg gctggtagcc ggaacacagt gtccagatgg gcagttctgc     120
   cctgttgcct gctgccttga ccagggagga gccaactaca gctgctgtaa ccctcttctg     180
   gacacatggc ctagaataac gagccatcat ctagatggct cctgccagac ccatggccac     240
   tgtcctgctg gctattcttg tcttctcact gtgtctggga cttccagctg ctgcccgttc     300
   tctaagggtg tgtcttgtgg tgatggctac cactgctgcc cccagggctt ccactgtagt     360
   gcagatggga aatcctgctt ccagatgtca gataacccct tgggtgctgt ccagtgtcct     420
   gggagccagt ttgaatgtcc tgactctgcc acctgctgca ttatggttga tggttcgtgg     480
   ggatgttgtc ccatgcccca ggcctcttgc tgtgaagaca gagtgcattg ctgtccccat     540
   ggggcctcct gtgacctggt tcacacacga tgcgtttcac ccacgggcac ccacacccta     600
   ctaaagaagt tccctgcaca aaagaccaac agggcagtgt ctttgccttt ttctgtcgtg     660
   tgccctgatg ctaagaccca gtgtcccgat gattctacct gctgtgagct acccactggg     720
   aagtatggct gctgtccaat gcccaatgcc atctgctgtt ccgaccacct gcactgctgc     780
   ccccaggaca ctgtatgtga cctgatccag agtaagtgcc tatccaagaa ctacaccacg     840
   gatctcctga ccaagctgcc tggataccca gtgaaggagg tgaagtgcga catggaggtg     900
   agctgccctg aaggatatac ctgctgccgc ctcaacactg gggcctgggg ctgctgtcca     960
   tttgccaagg ccgtgtgttg tgaggatcac attcattgct gcccggcagg gtttcagtgt    1020
   cacacagaga aaggaacctg cgaaatgggt atcctccaag taccctggat gaagaaggtc    1080
   atagcccccc tccgcctgcc agacccacag atcttgaaga gtgatacacc ttgtgatgac    1140
   ttcactaggt gtcctacaaa caatacctgc tgcaaactca attctgggga ctggggctgc    1200
   tgtcccatcc cagaggctgt ctgctgctca gacaaccagc attgctgccc tcagggcttc    1260
   acatgtctgg ctcaggggta ctgtcagaag ggagacacaa tggtggctgg cctggagaag    1320
   atacctgccc gccagacaac cccgctccaa attggagata tcggttgtga ccagcatacc    1380
   agctgcccag tagggcaaac ctgctgccca agcctcaagg gaagttgggc ctgctgccag    1440
   ctgccccatg ctgtgtgctg tgaggaccgg cagcactgtt gcccggccgg gtacacctgc 1500
   aatgtgaagg cgaggacctg tgagaaggat gtcgatttta tccagcctcc cgtgctcctg 1560
   accctcggcc ctaaggttgg gaatgtggag tgtggagaag ggcatttctg ccatgataac 1620
   cagacctgtt gtaaagacag tgcaggagtc tgggcctgct gtccctacct aaagggtgtc 1680
   tgctgtagag atggacgtca ctgttgcccc ggtggcttcc actgttcagc caggggaacc 1740
   aagtgtttgc gaaagaagat tcctcgctgg gacatgtttt tgagggatcc ggtcccaaga 1800
   ccgctactgt aaggaagggc tacagactta aggaactcca cagtcctggg aaccctgttc 1860
   cgagggtacc cactactcag gcctccctag cgcctcctcc cctaacgtct ccccggccta 1920
   ctcatcctga gtcaccctat caccatggga ggtggagcct caaactaaaa ccttctttta 1980
   tggaaagaag gctgtggcca aaagccccgt atcaaactgc catttcttcc ggtttctgtg 2040
   gaccttgtgg ccaggtgctc ttcccgagcc acaggtgttc tgtgagcttg cttgtgtgtg 2100
   tgtgcgcgtg tgcgtgtgtt gctccaataa agtttgtaca ctttc 2145
<210> 14
   <211> 25
   <212> DNA
   <213> Artificial Sequence
<220>
   <223> Description of Artificial Sequence: Synthetic oligonucleotide
<400> 14
   gagcaggtgg atttgtgaac agcgg 25
<210> 15
   <211> 25
   <212> DNA
   <213> Artificial Sequence
<220>
   <223> Description of Artificial Sequence: Synthetic oligonucleotide
<400> 15
   gaacacgtgg atttctgaag agagg 25
<210> 16
   <211> 25
   <212> DNA
   <213> Artificial Sequence
<220>
   <223> Description of Artificial Sequence: Synthetic oligonucleotide
<400> 16
   cctcttacct cagttacaat ttata 25
<210> 17
   <211> 22
   <212> DNA
   <213> Artificial Sequence
<220>
   <223> Description of Artificial Sequence: Synthetic oligonucleotide
<400> 17
   cgacatcttc tgcaccattg gc 22
<210> 18
   <211> 25
   <212> DNA
   <213> Artificial Sequence
<220>
   <223> Description of Artificial Sequence: Synthetic oligonucleotide
<400> 18
   tttaaatatt tcccaagtcc aacgt 25
<210> 19
   <211> 6
   <212> PRT
   <213> Artificial Sequence
<220>
   <223> Description of Artificial Sequence: Synthetic 6xHis tag
<400> 19
   His His His His His His
   1               5

## Description

### FIELD OF THE INVENTION

This invention is directed to methods and compositions for treating neurodegenerative diseases. More particularly, the present disclosure is directed to methods of treatment of neurodegenerative diseases using progranulin and methods of treatment of neurodegenerative diseases using effectors, or combinations of effectors, that modify progranulin. The invention is defined in the claims.

### BACKGROUND AND SUMMARY

Progranulin (PGRN) is a growth factor-like protein that is involved in the regulation of multiple processes including development, wound healing, angiogenesis, growth and maintenance of neuronal cells, and inflammation. An increase in PGRN expression has been linked to tumor promotion. Additionally, altered PGRN expression has been shown in multiple neurodegenerative diseases, including Creutzfeldt-Jakob disease, motor neuron disease, and Alzheimer's disease. For example, recent studies into the genetic etiology of neurodegenerative diseases have shown that heritable mutations in the PGRN gene may lead to adult-onset neurodegenerative diseases due to reduced neuronal survival.

Selective neuronal cell death is the common hallmark of various neurodegenerative disorders. Sporadic forms of Alzheimer's disease, Parkinson's disease, and Lou Gehrig's disease (amyotrophic lateral sclerosis (ALS)) have been linked to environmental factors that cause neuronal cell death by excitotoxicity, oxidative stress, inhibition of parts of the electron transport chain, cellular and mitochondrial membrane disruption, alterations in cellular organelles, alterations in chromatin, general and specific genotoxic action, and inhibition and/or hyperactivation of cell surface protein receptors and effectors. The experimental and clinical literature supports a potential role for excitotoxins in some forms of neurodegeneration, notably ALS and Alzheimer's disease. In particular, abnormalities in glutamate handling/transport have been linked to ALS and domoic acid, a kainate receptor (*i*.*e*., an ionotrophic glutamate receptor) agonist, has been shown to be a causal factor in some forms of memory loss, much like that reported in Alzheimer's disease. Oxidative stress has also been linked to the same disease states.

Toxins present in the environment may play a role in the pathology of various neurodegenerative diseases. For example, β-sitosterol β-D-glucoside (BSSG) has been identified as a toxin present in the seed of the cycad palm (*Cycas circinalis*), historically a staple of the diet of the Chamorro people of Guam. Cycad seed consumption has been linked to ALS-parkinsonism dementia complex (ALS-PDC), an endemic neurological disorder of Guam. *In vivo* studies in which adult male mice consume washed cycad seed flour as part of their diet have shown that treated animals have profound and progressive motor, cognitive, and olfactory behavioural deficits combined with the loss of neurons in each of the respective neural subsets. The expression of these outcomes mirrors the behavioural and pathological deficits in ALS-PDC. *In vitro* experiments using isolated cycad fractions have identified the likely neurotoxins as variant sterol glucoside molecules contained in washed cycad flour, specifically BSSG and related sterol glucoside molecules.

Currently, there is no cure for ALS, Alzheimer's disease (AD), or Parkinson's disease. Current treatment generally involves efforts by physicians to slow progression of the symptoms and make patients more comfortable. While there are a number of drugs in development and a limited number that are FDA approved for treatment (Riluzole, for ALS; L-dopa for Parkinson's disease; cognitive enhancers, such as Aricept, for AD) these treatments only mask the progression of neurologic disease and may act to marginally prolong the lives of some patients. Thus, there is a significant need for methods and compositions directed to treatment of neurodegenerative diseases.

In one illustrative embodiment, a method for treating a patient with a neurodegenerative disease is provided, the method comprising the steps of administering to the patient a composition comprising a progranulin polypeptide, and reducing the symptoms of the neurodegenerative disease in the patient.

In the above described embodiment, the amount of the progranulin polypeptide administered to the patient can be in the range of about 1 ng/kg of patient body weight to about 1 mg/kg of patient body weight, the amount of the progranulin polypeptide administered to the patient can be in the range of about 1 ng/kg of patient body weight to about 500 ng/kg of patient body weight, the amount of the progranulin polypeptide administered to the patient can be in the range of about 1 ng/kg of patient body weight to about 100 ng/kg of patient body weight, the composition comprising the progranulin polypeptide can be adapted for parenteral administration, the route of parenteral administration can be selected from the group consisting of intradermally, subcutaneously, intramuscularly, intraperitoneally, intravenously, intraventricularly, intrathecally, intracerebrally, and intracordally, the neurodegenerative disease state can be mediated by an environmental insult to the patient, the neurodegenerative disease state can be mediated by an excitotoxin, the excitotoxin can be a sterol glycoside, the sterol glycoside can be selected from the group consisting of beta-sitosterol-beta-D-glucoside and cholesterol glucoside, or analogs or derivatives thereof, the neurodegenerative disease can be selected from the group consisting of Parkinson's disease, Alzheimer's disease, and amyotrophic lateral sclerosis, the neurodegenerative disease can be Parkinson's disease, the neurodegenerative disease can be Alzheimer's disease, the neurodegenerative disease can be amyotrophic lateral sclerosis, and the progranulin polypeptide can have at least 95% homology with SEQ ID NO: 2.

In another illustrative embodiment, a pharmaceutical composition comprising therapeutically effective amounts of progranulin polypeptide and a pharmaceutically acceptable carrier therefor is provided, wherein the therapeutically effective amounts comprise amounts capable of reducing or preventing the symptoms of a neurodegenerative disease in a patient.

In the above described embodiment, the composition can be adapted for parenteral administration, the route of parenteral administration can be selected from the group consisting of intradermally, subcutaneously, intramuscularly, intraperitoneally, intravenously, intraventricularly, intrathecally, intracerebrally, and intracordally, the neurodegenerative disease state can be mediated by an environmental insult to the patient, the neurodegenerative disease state can be mediated by an excitotoxin, the excitotoxin can be a sterol glycoside, the sterol glycoside can be selected from the group consisting of beta-sitosterol-beta-D-glucoside and cholesterol glucoside, or analogs or derivatives thereof, the neurodegenerative disease can be selected from the group consisting of Parkinson's disease, Alzheimer's disease, and amyotrophic lateral sclerosis, the neurodegenerative disease can be Parkinson's disease, the neurodegenerative disease can be Alzheimer's disease, the neurodegenerative disease can be amyotrophic lateral sclerosis, the pharmaceutical composition can be in a parenteral dosage form, the dosage form can be adapted for parenteral administration by a route selected from the group consisting of intradermal, subcutaneous, intramuscular, intraperitoneal, intravenous, intraventricular, intrathecal, intracerebral, and intracordal, and the progranulin polypeptide can have at least 95% homology with SEQ ID NO: 2

In another illustrative embodiment, a method for reducing neuronal cell death in a patient is provided, the method comprising the steps of administering to a patient a therapeutically effective amount of a progranulin polypeptide wherein the amount of the peptide is effective to increase neuronal cell survival in a patient with a neurodegenerative disease, and reducing neuronal cell death in the patient.

In the above described embodiment, the amount of the progranulin polypeptide administered to the patient can be in the range of about 1 ng/kg of patient body weight to about 1 mg/kg of patient body weight, the amount of the progranulin polypeptide administered to the patient can be in the range of about 1 ng/kg of patient body weight to about 500 ng/kg of patient body weight, the amount of the progranulin polypeptide administered to the patient can be in the range of about 1 ng/kg of patient body weight to about 100 ng/kg of patient body weight, the composition comprising the progranulin polypeptide can be adapted for parenteral administration, the route of parenteral administration can be selected from the group consisting of intradermally, subcutaneously, intramuscularly, intraperitoneally, intravenously, intraventricularly, intrathecally, intracerebrally, and intracordally, the neurodegenerative disease state can be mediated by an environmental insult to the patient, the neurodegenerative disease state can be mediated by an excitotoxin, the excitotoxin can be a sterol glycoside, the sterol glycoside can be selected from the group consisting of beta-sitosterol-beta-D-glucoside and cholesterol glucoside, or analogs or derivatives thereof, the neurodegenerative disease can be selected from the group consisting of Parkinson's disease, Alzheimer's disease, and amyotrophic lateral sclerosis, the neurodegenerative disease can be Parkinson's disease, the neurodegenerative disease can be Alzheimer's disease, the neurodegenerative disease can be amyotrophic lateral sclerosis, and the progranulin polypeptide can be a polypeptide wherein the polypeptide has at least 95% homology with SEQ ID NO: 2.

In another illustrative embodiment, a method for treating a patient with a neurodegenerative disease is provided, the method comprising the steps of administering to the patient a composition comprising an effector that modifies progranulin expression, and reducing the symptoms of the neurodegenerative disease in the patient.

In the above described embodiment, the composition comprising the effector can be adapted for parenteral administration, the route of parenteral administration can be selected from the group consisting of intradermally, subcutaneously, intramuscularly, intraperitoneally, intravenously, intraventricularly, intrathecally, intracerebrally, and intracordally, the neurodegenerative disease can be mediated by an environmental insult to the patient, the neurodegenerative disease state can be mediated by an excitotoxin, the excitotoxin can be a sterol glycoside, the sterol glycoside can be selected from the group consisting of beta-sitosterol-beta-D-glucoside and cholesterol glucoside, or analogs or derivatives thereof, the neurodegenerative disease can be selected from the group consisting of Parkinson's disease, Alzheimer's disease, and amyotrophic lateral sclerosis, the neurodegenerative disease can be Parkinson's disease, the neurodegenerative disease can be Alzheimer's disease, the neurodegenerative disease can be amyotrophic lateral sclerosis, the method can further comprise the step of increasing the expression of progranulin in neurons, the method can further comprise the step of decreasing the expression of progranulin in non-neuronal cells, and the effector can be a vector comprising a nucleic acid with at least 95% homology with SEQ ID NO: 1.

In another illustrative embodiment, a pharmaceutical composition comprising therapeutically effective amounts of an effector that modifies progranulin expression and a pharmaceutically acceptable carrier therefor is provided, wherein the therapeutically effective amounts comprise amounts capable of reducing or preventing the symptoms of a neurodegenerative disease in a patient.

In the above described embodiment, the therapeutically effective amounts can comprise amounts capable of increasing progranulin expression in neurons, the therapeutically effective amounts can comprise amounts capable of decreasing progranulin expression in non-neuronal cells, the pharmaceutical composition can be in a parenteral dosage form, the dosage form can be adapted for parenteral administration by a route selected from the group consisting of intradermal, subcutaneous, intramuscular, intraperitoneal, intravenous, intraventricular, intrathecal, intracerebral, and intracordal, the neurodegenerative disease state can be mediated by an environmental insult to the patient, the neurodegenerative disease state can be mediated by an excitotoxin, the excitotoxin can be a sterol glycoside, the sterol glycoside can be selected from the group consisting of beta-sitosterol-beta-D-glucoside and cholesterol glucoside, or analogs or derivatives thereof, the neurodegenerative disease can be selected from the group consisting of Parkinson's disease, Alzheimer's disease, and amyotrophic lateral sclerosis, the neurodegenerative disease can be Parkinson's disease, the neurodegenerative disease can be Alzheimer's disease, the neurodegenerative disease can be amyotrophic lateral sclerosis, the pharmaceutical composition can be in a parenteral dosage form, the dosage form can be adapted for parenteral administration by a route selected from the group consisting of intradermal, subcutaneous, intramuscular, intraperitoneal, intravenous, intraventricular, intrathecal, intracerebral, and intracordal, and the effector can be a vector comprising a nucleic acid with at least 95% homology with SEQ ID NO: 1.

In another illustrative embodiment, a method for reducing neuronal cell death in a patient is provided, the method comprising the steps of administering to a patient a therapeutically effective amount of an effector that modifies progranulin expression wherein the amount of the effector is effective to increase neuronal cell survival in a patient with a neurodegenerative disease mediated by an environmental insult to the patient, and reducing neuronal cell death in the patient.

In the above described embodiment, the composition comprising the effector can be adapted for parenteral administration, the route of parenteral administration can be selected from the group consisting of intradermally, subcutaneously, intramuscularly, intraperitoneally, intravenously, intraventricularly, intrathecally, intracerebrally, and intracordally, the neurodegenerative disease state can be mediated by an environmental insult to the patient, the neurodegenerative disease state can be mediated by an excitotoxin, the excitotoxin can be a sterol glycoside, the sterol glycoside can be selected from the group consisting of beta-sitosterol-beta-D-glucoside and cholesterol glucoside, or analogs or derivatives thereof, the neurodegenerative disease can be selected from the group consisting of Parkinson's disease, Alzheimer's disease, and amyotrophic lateral sclerosis, the neurodegenerative disease can be Parkinson's disease, the neurodegenerative disease can be Alzheimer's disease, the neurodegenerative disease can be amyotrophic lateral sclerosis, and the effector can be a vector comprising a nucleic acid with at least 95% homology with SEQ ID NO: 1

### BRIEF DESCRIPTION OF THE DRAWINGS

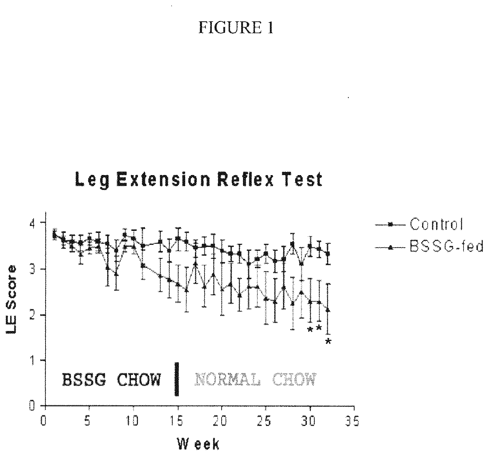

Figure 1 shows a graph indicating a progressive decrease in leg extension reflex in mice following synthetic steryl glucoside (BSSG) exposure.

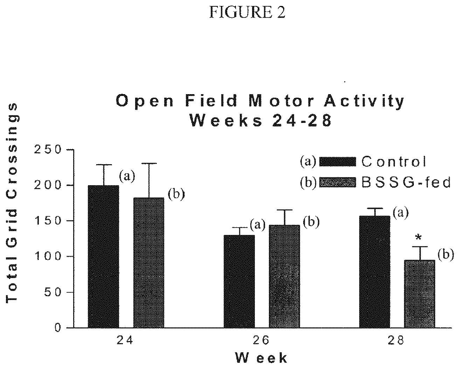

Figure 2 shows a graph indicating a progressive decrease in open field motor activity in mice following synthetic steryl glucoside (BSSG) exposure.

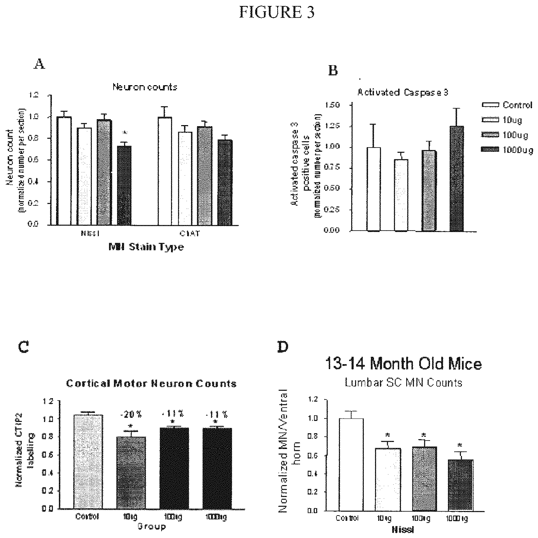

Figure 3 A-D shows graphs indicating a progressive loss of motor neurons in mice following 10 weeks of exposure to synthetic steryl glucoside (BSSG). Following BSSG exposure, mice were allowed to age for 1 month (panels A-C) or 5 months (panel D) with a diet of normal chow prior to sacrifice. Panel A shows the quantification of motor neurons in lumbar cord following Nissl staining and choline acetyltransferasc immunohistochemistry (ChAT). Panel B shows the quantification of activated caspase-3 in lumbar spinal cord following detection by immunohistochemistry (ventral horn). Panel C shows the quantification of neurons in the motor cortex following detection by immunohistochemistry against CTIP2 (highly expressed in corticospinal motor neurons). Panel D shows the quantification of motor neurons in lumbar cord following Nissl staining.

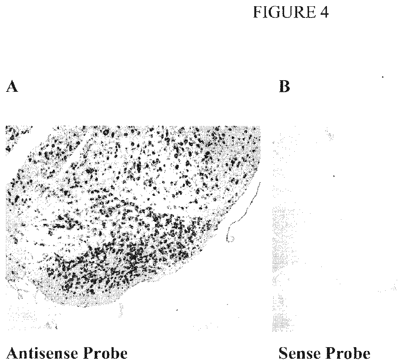

Figure 4 shows *in situ* hybridization (ISH) to detect progranulin expression in the brainstem of a normal mouse (original magnification 10X). Panel A: ISH using antisense, progranulin specific riboprobes. Panel B: ISH using sense riboprobes.

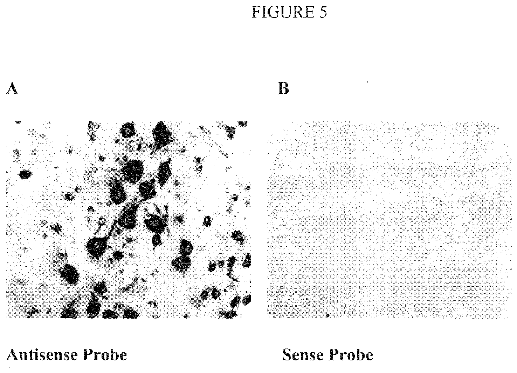

Figure 5 shows *in situ* hybridization (ISH) to detect progranulin expression in the brainstem of a normal mouse at high magnification (original magnification 40X). Panel A: ISH using antisense, progranulin specific riboprobes. Panel B: ISH using sense riboprobes.

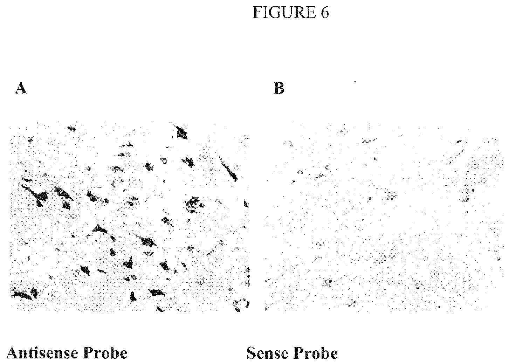

Figure 6 shows *in situ* hybridization (ISH) to detect progranulin expression in anterior horn cells of the spinal cord of a normal mouse (original magnification 40X). Panel A: ISH using antisense, progranulin specific riboprobes. Panel B: ISH using sense riboprobes.

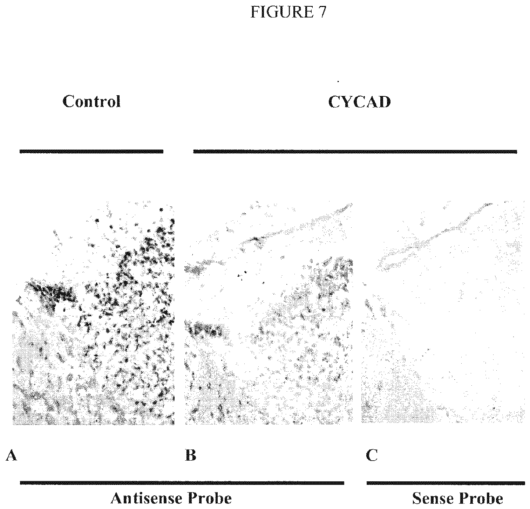

Figure 7 shows *in situ* hybridization (ISH) indicating decreased progranulin expression in the motor cortex of washed cycad flour-fed mice (original magnification 10 X). Panel A: ISH using antisense, progranulin specific riboprobes on the motor cortex of normal chow-fed mice. Panel B: ISH using antisense, progranulin specific riboprobes on the motor cortex of cycad flour-fed mice. Panel C: ISH using sense riboprobes on the motor cortex of cycad flour-fed mice.

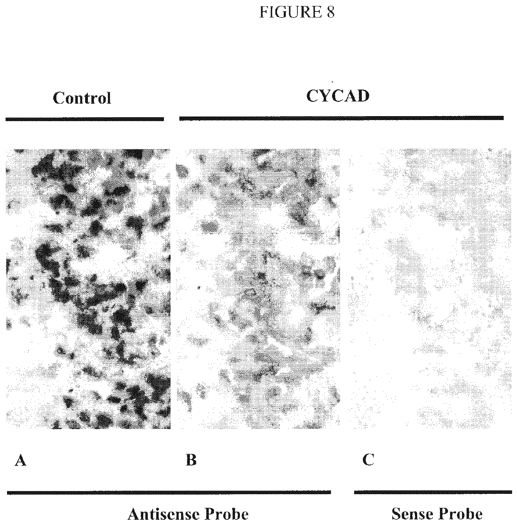

Figure 8 shows *in situ* hybridization (ISH) indicating decreased progranulin expression in the motor cortex of washed cycad flour-fed mice (original magnification 40 X). Panel A: ISH using antisense, progranulin specific riboprobes on the motor cortex of normal chow-fed mice. Panel B: ISH using antisense, progranulin specific riboprobes on the motor cortex of cycad flour-fed mice. Panel C: ISH using sense riboprobes on the motor cortex of cycad flour-fed mice.

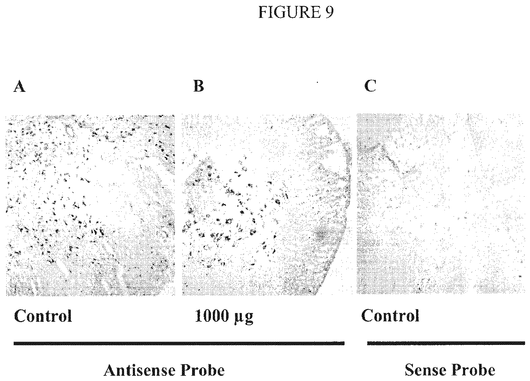

Figure 9 shows *in situ* hybridization (ISH) indicating that exposure to synthetic BSSG results in decreased progranulin expression in the anterior horn cells of the cervical spinal cord of mice. Panel A: ISH using antisense, progranulin specific riboprobes on the cervical spinal cord of normal chow-fed mice. Panel B: ISH using antisense, progranulin specific riboprobes on the cervical spinal cord of mice fed 1000 µg/day BSSG. Panel C: ISH using sense riboprobes on the cervical spinal cord of normal chow-fed mice (original magnification 10X).

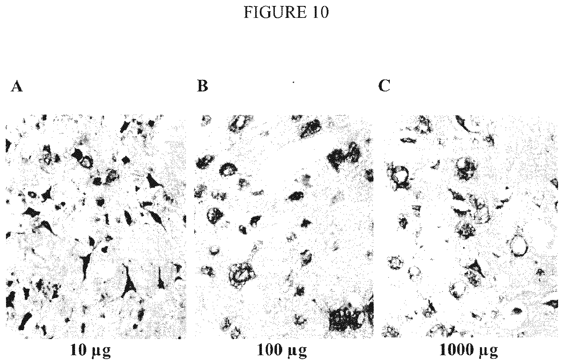

Figure 10 shows *in situ* hybridization (ISH) indicating that increasing BSSG exposure in mice results in more pronounced neuropathology and loss of progranulin expression in the anterior horn cells of the cervical spinal cord. Panels A, B, and C show ISH using antisense, progranulin specific riboprobes on the cervical spinal cord of mice fed 10, 100, and 1000 µg/day BSSG, respectively (original magnification 40X).

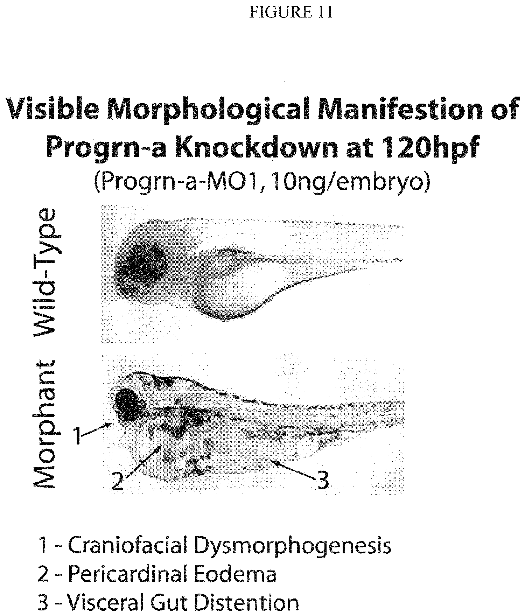

Figure 11 shows that a knockdown of progranulin expression in zebrafish leads to the morphological manifestations of craniofacial dysmorphogenesis, pericardial edema, and visceral gut distention.

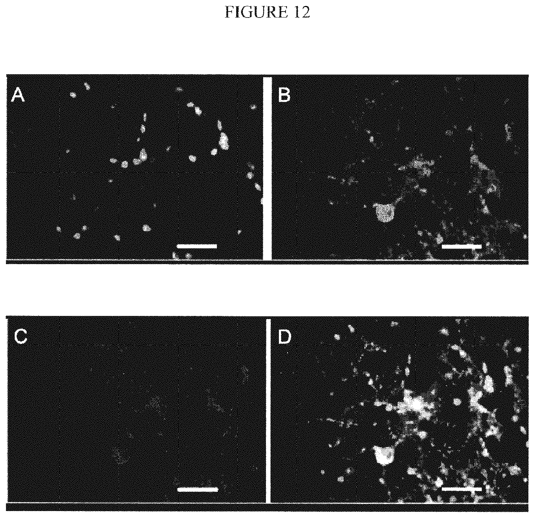

Figure 12 shows progranulin expressed by heterogenous neurons in vitro. Primary neuronal cultures were derived from an E13 mouse embryo spinal cord. Immunolocalization of nuclei (DAPI; Panel A), non-phosphorylated neurofilament using SMI32 (Panel B) and mouse progranulin (Panel C). Primary motor neurons that express progranulin were identified based on cell body size and SMI32 immunoreactivity (Panels B-D). Progranulin was found throughout motor neurons except for the nucleus. (Panel D) Merged channels from all three fluorophores. Scale bar represents 20um.

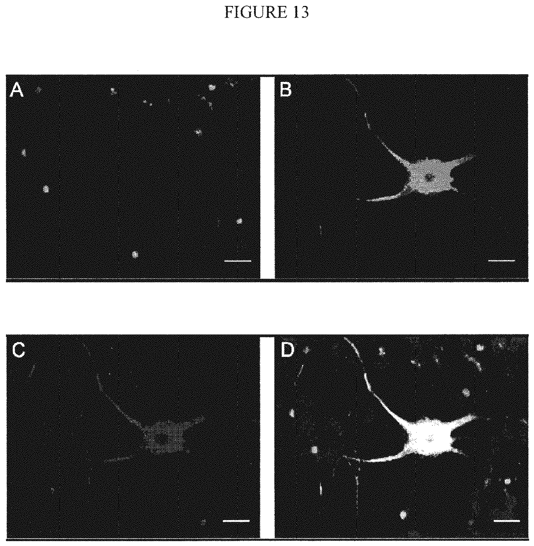

Figure 13 shows progranulin expressed by mouse motor neurons in vivo. Motor neurons within the lumbar spinal cord of 8 week old CD-1 mice were examined. Immunofluorescence of the dorsal horn portion of the lumbar spinal for DAPI (Panel A), non-phosphorylated neurofilament using the SMI32 antibody (Panel B) and progranulin (Panel C). (Panel D) Merged channels from all three fluorophores. Scale bar represents 30um.

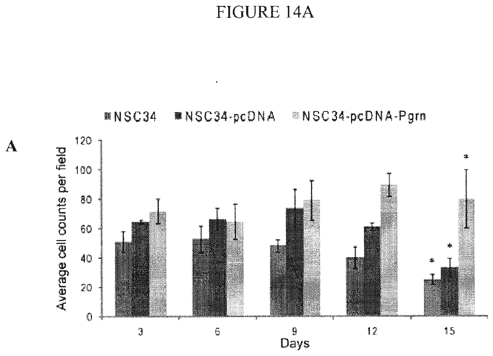

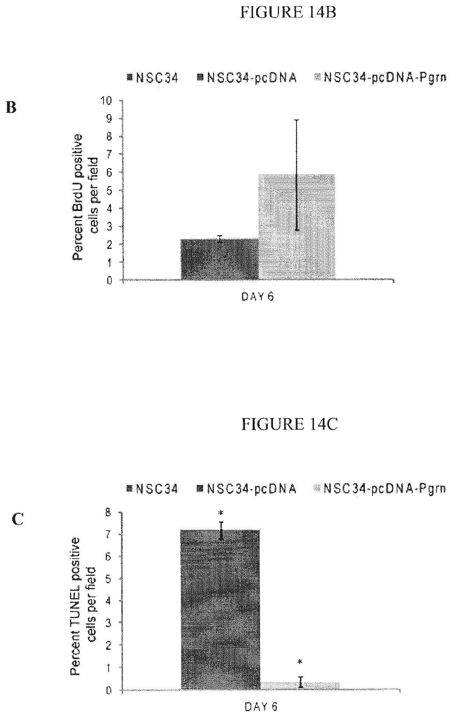

Figure 14 shows progranulin overexpression resulting in cell survival in serum free medium. Untransfected NSC34 cells (first bar), stable vector only transfectants (NSC34-pcDNA; middle bar) and stable progranulin overexpressing cells (NSC34-pcDNA-Pgrn; last bar) were cultured in serum free RPMI medium. (Panel A). Average cell counts per field (10x magnification) were determined at three day intervals by phase-contrast microscopy for fifteen days. Progranulin over expressing cells demonstrate increased survival as compared to controls (Asterisks denote P<0.005). (Panel B) Cell proliferation assay based on 12 hour BrdU incorporation following 6 days culture in serum free medium. Progranulin overexpression (second bar) during serum deprivation does not significantly increase cell proliferation rates (P>0.1). (Panel C). Apoptosis assay based on the TUNEL labeling method following 6 days in serum free medium. Progranulin overexpression (second bar) during serum deprivation protects against apoptosis (Asterisks denote P<0.0001, Two tailed Student's T test).

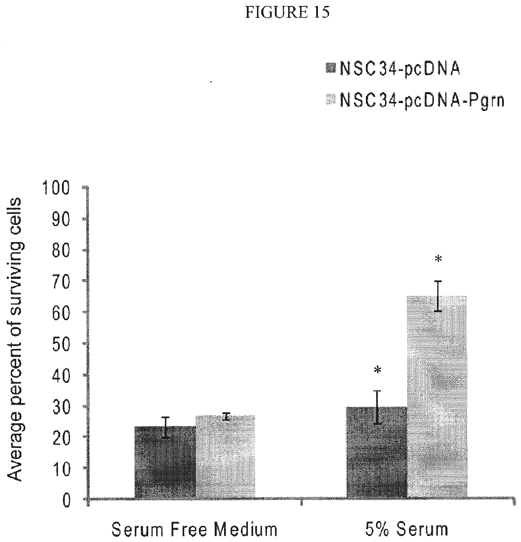

Figure 15 shows that progranulin is a key trophic factor for cell survival during chronic hypoxia. The NSC34 cell line was stably transfected with vector only (pcDNA; first bar) or human progranulin (pcDNA-Pgrn; second bar). Cells were cultured in serum free medium or 5% serum within an atmosphere consisting of 1% oxygen (80% reduced oxygen tension) for 72 hours and remaining cells were counted using a hemocytometer (Asterisks denote P<0.0001).

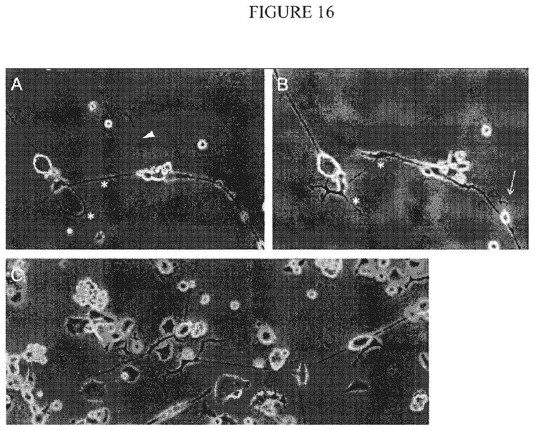

Figure 16 shows that progranulin overexpression results in dynamic neuronal pathfinding and cell survival in NSC34 cells. (Panels A-C) Phase contrast microscopy of NSC34 cells stably transfected with pcDNA3.1/Pgrn. (Panels A and B) Time lapse phase-contrast micrographs over a single 3 hour period demonstrating active extension (arrow), retraction (arrowhead) and gross rearrangements (asterisks) of neuritic processes, following maintenance of the cultures in serum free medium for 20 days. (Panel C) Continued survival and maintenance of a neural-like morphology following 57 days in serum free medium.

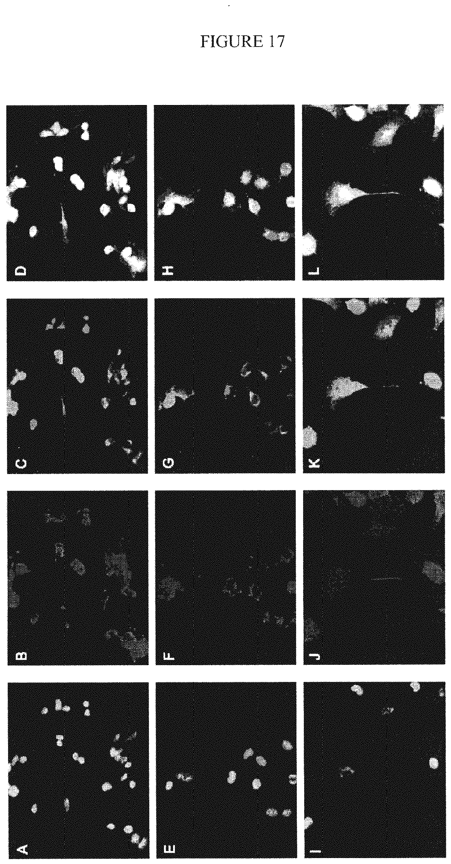

Figure 17 shows that progranulin overexpression results in the development of neuronal morphology in NSC-34 cells. Immunofluorescent images of nuclei [DAPI] (Panels A, E, and I), F-actin [Phalloidin stain] (Panels B, F, and J), Progranulin-IHC (Panels C, G, and H) and merged [nuclear/actin/progranulin] (Panels D, H, and L). Untransfected control NSC-34 cells (panels A-D), mock transfected NSC-34 cells (Panels E-H) and progranulin overexpressing cells (Panels I-H) are depicted. Note the more extensive cytoplasm with process extensions in the progranulin overexpressing cells (Panels J-L). Original magnification 43x.

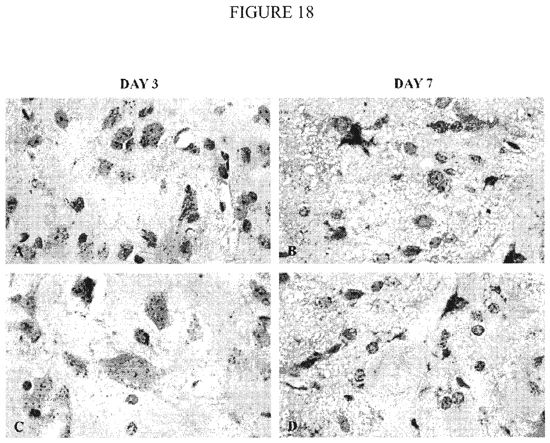

Figure 18 shows progranulin expression following acute neuronal stress. At day 3 post-axotomy, abundant Progranulin immunoreactivity is observed within motor neurons with very little evidence of non-neuronal staining (Panel A). The pattern of immunostaining is punctate and diffusely distributed throughout the cytosol (Panel C). At day 7, in contrast, immunostaining is now intensely seen in the microglial cells (Panel B), while the motor neurons are devoid of Progranulin staining (Panel D).

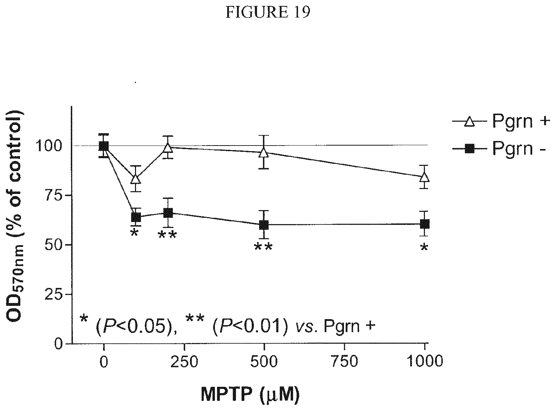

Figure 19 shows the protective effect of progranulin against the neurotoxin MPTP in PC-12 cells.

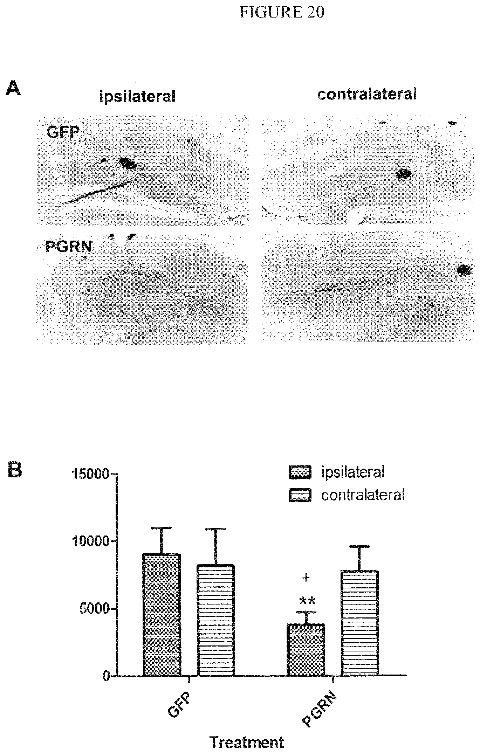

Figure 20 shows the immunohistochemical analysis of amyloid burden following intracerebral viral vector delivery of either GFP or PGRN in aged Tg2576 mice. Panel A shows representative photomicrographs depicting coronal sections through the hippocampus immunostained for β-amyloid (AB), following either lentiviral-GFP or lentiviral-PGRN treatment. Panel B shows the quantitative evaluation of changes in Aβ load. In animals receiving lentiviral-PGRN, a significant decline in amyloid burden was evident in the ipsilateral hippocampus (F_{2,47} = 5.86621, *p* < 0.0095, TREATMENT main effect). Each bar represents the mean (± S.E.M.) (*n* = 8) area (µm²) occupied by amyloid immunolabeling, as measured in 3 sections through the hippocampus (** sig. diff. from lentiviral-GFP control, *p* < 0.001)(+ sig. diff. from contralateral hemisphere, *p* < 0.05).

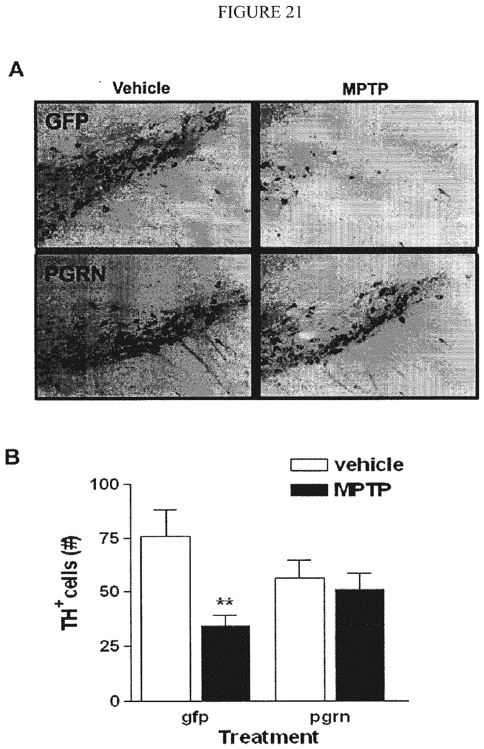

Figure 21 shows the immunohistochemical analysis of MPTP effects on TH⁺ cell counts following intracerebral viral vector delivery of either lentiviral(LV)-GFP or lentiviral-PGRN. Panel A shows representative fluorescent photomicrographs depicting coronal sections through the SNc immunostained for TH, following MPTP intoxication. Panel B shows the quantitative evaluation of TH⁺ cell counts in the SNc. In animals receiving LV-GFP, MPTP exposure resulted in a significant reduction in TH⁺ cell counts (Student t-test, *p* = 0.0041), while no significant loss of cells was observed in those animals receiving LV-PGRN (*p* = 0.64). Each bar represents the mean (± S.E.M.) (*n* = 4-6) number of TH⁺ cells measured in 3 sections through the SNc (** sig. diff. from vehicle-treated controls, *p* < 0.001).

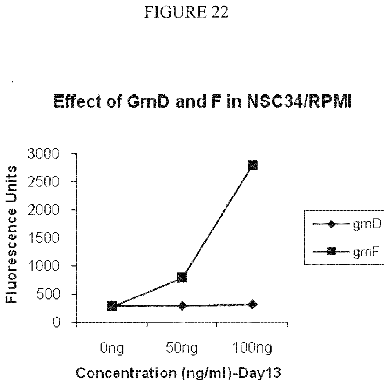

Figure 22 shows the immortalized motor neuron cell line (NSC-34) incubated with grn F and grn D with either proliferation/survival (grn F) or no effect (grn D).

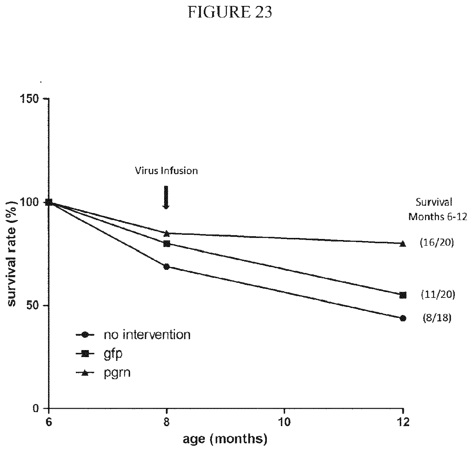

Figure 23 shows survival of Tg2576 following intracerebral delivery of either GFP or PGRN expressing lentiviruses.

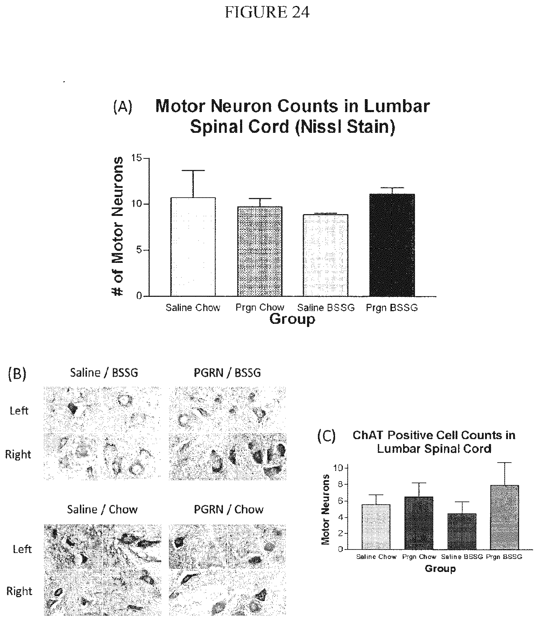

Figure 24 shows spinal motor neuron counts and choline acetyl transferase activity in PGRN lentivirus treated mice. Panel (A) shows motor neuron counts assessed by Nissl stain. Panel B shows the immunohistolochemical assessment of choline acetyl transferase (ChAT) activity in anterior horn cells of saline treated BSSG exposed mice relative to all other treatment groups (upper right hand panels of (B). Images in (B) are representative and are taken from both left and right anterior horns (initial magnification 100x). Panel C shows ChAT positive motor neuron in control (saline treated) and BSSG exposed mice.

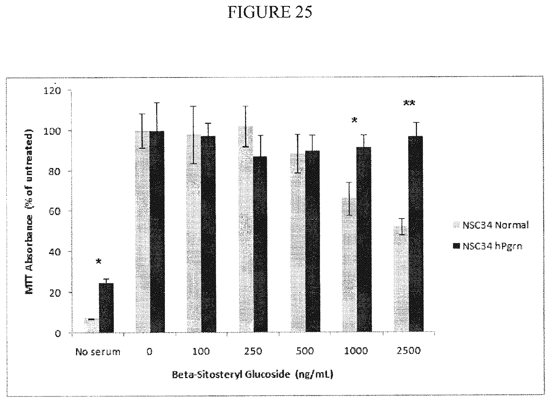

Figure 25 shows Beta-Sitosteryl Glucoside (ng/ml) plotted against MTT Absorbance for normal NSC-34 cells and a stable transfectant that over-expresses human progranulin (NSC-34 hPGRN). Cells were plated at 8,000cells/well and maintained in DMEM 5% FCS for 72 hours in the presence of various concentration of BSSG. As a negative control for cell proliferation/survival a series of wells were cultured without BSSG or serum. Standard error bars along with Pvalues of <0.05 (*) or <0.001 (**) are illustrated.

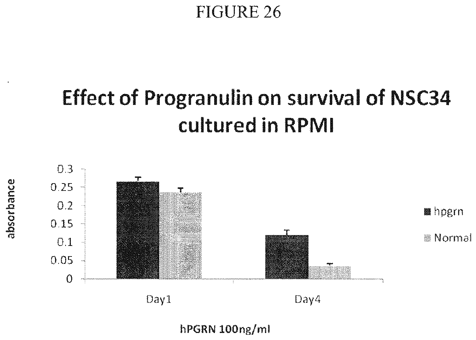

Figure 26 shows hPRRN (100 ng/ml) plotted against absorbance. Human recombinant progranulin (PGRN) protein added to NSC-34 cells in culture resulted in a 2. 5 fold (Day 4) increase in cell survival following serum starvation. Error bars represent standard deviation of the mean. Absorbance was measured at 570nM.

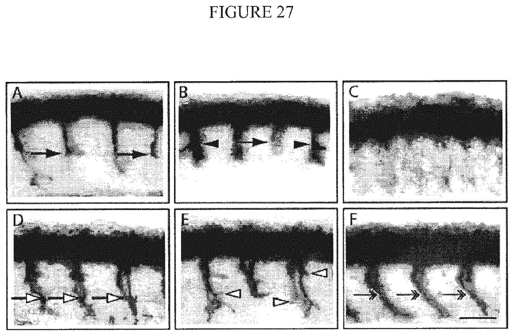

Figure 27 shows lateral views (anterior to the left; dorsal to the top) of whole-mount embryos immuonolabelled with znp1 mAb at 27 hpf (Panels A-F). Panels A, B, C: embryos injected with progranulin MO. Panels D, E: embryos injected with progranulin-A-MO+100pg progranulin-A mRNA. Panel F: wild type. Truncated or complete loss of motor axon nerves (Panels A and B; black arrows and Panel C). Branched motor axons/nerves (Panel B; black arrowheads), partial rescue of motor axons (Panel D; white arrows) and increase in branched axons/nerves (Panel E; white arrowheads) occur.

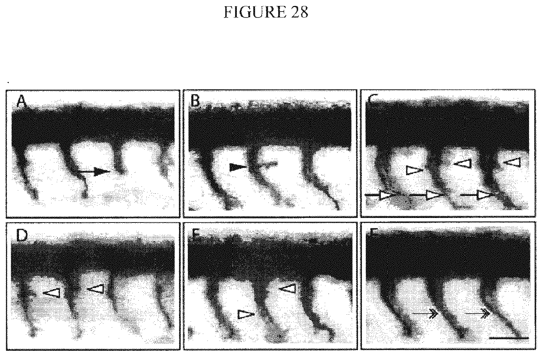

Figure 28 shows lateral views (anterior to the left; dorsal to the top) of whole-mount embryos labeled with znp1 mAb at 27 hpf (Panels A-F) in embryos injected with smn1 MO(Panels A and B), smn1 MO+100pg progranulin-A mRNA co-injected (Panel C), 100pg progranulin-A mRNA (Panels D and E) or wild type (Panel F). Truncated motor axon nerves (Panel A; black arrows), branched motor axons/nerves (Panel B; black arrowheads), rescue of motor axons/nerves (Panel C; white arrow) and increase in branched axons/nerves (Panel C; white arrowheads), branched axons/nerves (Panel D, E; white arrowheads) and normal motor axons (Panel F; double arrowhead) occur. Scale Bar= 50µm.

### DETAILED DESCRIPTION

Methods and compositions are provided for treating a neurodegenerative disease mediated by an environmental insult to a patient. In one illustrative embodiment, patients with a neurodegenerative disease can be treated by administering to the patient a composition comprising a progranulin, wherein treatment of the patient with the composition comprising a progranulin reduces the symptoms of the neurological disease in the patient.

In the above described illustrative embodiment, the neurodegenerative disease can be mediated by environmental insult.

In another embodiment, a method is provided for reducing neuronal cell death in a patient. The method comprises the steps of administering to a patient with a neurodegenerative disease a therapeutically effective amount of a progranulin, wherein the amount of a progranulin is effective to increase neuronal cell survival or proliferation in the patient.

In the above described illustrative embodiment, the neurodegenerative disease can be mediated by environmental insult.

As used herein "a progranulin" or "a progranulin polypeptide" refers to a polypeptide selected from a polypeptide of SEQ ID NO. 2, a polypeptide with about 95% homology, about 96%, about 97%, about 98%, about 99% homology with SEQ ID NO. 2; a polypeptide of SEQ ID NO. 12, a polypeptide with about 95% homology, about 96%, about 97%, about 98%, about 99% homology with SEQ ID NO. 12; a polypeptide of SEQ ID NO. 3, a polypeptide with about 95% homology, about 96%, about 97%, about 98%, about 99% homology with SEQ ID NO. 3; a polypeptide of SEQ ID NO. 4, a polypeptide with about 95% homology, about 96%, about 97%, about 98%, about 99% homology with SEQ ID NO. 4; a polypeptide of SEQ ID NO. 5, a polypeptide with about 95% homology, about 96%, about 97%, about 98%, about 99% homology with SEQ ID NO. 5; a polypeptide of SEQ ID NO. 6, a polypeptide with about 95% homology, about 96%, about 97%, about 98%, about 99% homology with SEQ ID NO. 6; a polypeptide of SEQ ID NO. 7, a polypeptide with about 95% homology, about 96%, about 97%, about 98%, about 99% homology with SEQ ID NO. 7; a polypeptide of SEQ ID NO. 8, a polypeptide with about 95% homology, about 96%, about 97%, about 98%, about 99% homology with SEQ ID NO. 8; or a polypeptide of SEQ ID NO. 9, a polypeptide with about 95% homology, about 96%, about 97%, about 98%, about 99% homology with SEQ ID NO. 9.

Human Progranulin SEQ ID NO: 2

Human Progranulin DNA SEQ ID NO: 1

Mouse Progranulin SEQ ID NO: 12

Mouse Progranulin DNA SEQ ID NO: 13

SEQ ID NO: 3 hGmA

SEQ ID NO: 4 hGrnB

SEQ ID NO: 5 hGrnC

-VPCDNVSS-CPSSDTCCQLTSGEWGCCPIPEAVCCSDHQHCCPQGYTCVAEGQ-CQ

SEQ ID NO: 6 hGrnD

### SEQ ID NO: 7 hGrnE

SEQ ID NO: 8 hGrnF

AIQCPDSQFECPDFSTCCVMVDGSWGCCPMPQASCCEDRVHCCPHGAFCDLVHTRCI

SEQ ID NO: 9 hGrnG

GGPCQVDAH-CSAGHSCIFTVSGTSSCCPFPEAVACGDGHHCCPRGFHCSADGRSCF

SEQ ID NO: 10 grn D

SEQ ID NO: 11 grn F

As is well known to those skilled in the art, altering any non-critical amino acid of a protein by conservative substitution should not significantly alter the activity of that protein because the side-chain of the amino acid which is used to replace the natural amino acid should be able to form similar bonds and contacts as the side chain of the amino acid which has been replaced.

Non-conservative substitutions are possible provided that these do not excessively affect the neuroprotective or neuroregenerative activity of the polypeptide and/or reduce its effectiveness in treating neurodenerative diseases.

As is well-known in the art, a "conservative substitution" of an amino acid or a "conservative substitution variant" of a polypeptide refers to an amino acid substitution which maintains: 1) the structure of the backbone of the polypeptide (e.g. a beta sheet or alpha-helical structure); 2) the charge or hydrophobicity of the amino acid; and 3) the bulkiness of the side chain or any one or more of these characteristics. More specifically, the well-known terminologies "hydrophilic residues" relate to serine or threonine. "Hydrophobic residues" refer to leucine, isoleucine, phenylalanine, valine or alanine. "Positively charged residues" relate to lysine, arginine or histidine. "Negatively charged residues" refer to aspartic acid or glutamic acid. Residues having "bulky side chains" refer to phenylalanine, tryptophan or tyrosine.

The terminology "conservative amino acid substitutions" is well known in the art, which relates to substitution of a particular amino acid by one having a similar characteristic (e.g., similar charge or hydrophobicity, similar bulkiness). Examples include aspartic acid for glutamic acid, or isoleucine for leucine. A list of illustrative conservative amino acid substitutions is given in TABLE 1. A conservative substitution variant will 1) have only conservative amino acid substitutions relative to the parent sequence, 2) will have at least 90% sequence identity with respect to the parent sequence, preferably at least 95% identity, 96% identity, 97% identity, 98% identity or 99% or greater identity; and 3) will retain neuroprotective or neurorestorative activity. In this regard, any conservative substitution variant of the above-described polypeptide sequences is contemplated in accordance with this invention. Such variants are considered to be "a progranulin."

**TABLE 1**

| For Amino Acid | Replace With |
|---|---|
| Alanine | D-Ala, Gly, Aib, β-Ala, L-Cys, D-Cys |
| Arginine | D-Arg, Lys, D-Lys, Orn D-Orn |
| Asparagine | D-Asn, Asp, D-Asp, Glu, D-Glu Gln, D-Gln |
| Aspartic Acid | D-Asp, D-Asn, Asn, Glu, D-Glu, Gln, D-Gln |
| Cysteine | D-Cys, S-Me-Cys, Met, D-Met, Thr, D-Thr |
| Glutamine | D-Gln, Asn, D-Asn, Glu, D-Glu, Asp, D-Asp |
| Glutamic Acid | D-Glu, D-Asp, Asp, Asn, D-Asn, Gln, D-Gln |
| Glycine | Ala, D-Ala, Pro, D-Pro, Aib, β-Ala |
| Isoleucine | D-Ile, Val, D-Val, Leu, D-Leu, Met, D-Met |
| Leucine | Val, D-Val, Met, D-Met, D-Ile, D-Leu, Ile |
| Lysine | D-Lys, Arg, D-Arg, Orn, D-Orn |
| Methionine | D-Met, S-Me-Cys, Ile, D-Ile, Leu, D-Leu, Val, D-Val |
| Phenylalanine | D-Phe, Tyr, D-Tyr, His, D-His, Trp, D-Trp |
| Proline | D-Pro |
| Serine | D-Ser, Thr, D-Thr, allo-Thr, L-Cys, D-Cys |
| Threonine | D-Thr, Ser, D-Ser, allo-Thr, Met, D-Met, Val, D-Val |
| Tyrosine | D-Tyr, Phe, D-Phe, His, D-His, Trp, D-Trp |
| Valine | D-Val, Leu, D-Leu, Ile, D-Ile, Met, D-Met |

In one illustrative aspect, the neurodegenerative disease state can include, but is not limited to, Parkinson's disease and the parkinsonisms including progressive supranuclear palsy, Alzheimer's disease, and motor neuron disease (e.g., amyotrophic lateral sclerosis); or any other neurodegenerative disease mediated by an increase in neuronal cell death and a modification of progranulin expression.

In another embodiment, a pharmaceutical composition is provided. The pharmaceutical composition comprises therapeutically effective amounts of progranulin and a pharmaceutically acceptable carrier, wherein the therapeutically effective amounts comprise amounts capable of reducing or preventing the symptoms of a neurodegenerative disease mediated by an environmental insult to a patient.

The unitary daily dosage of the composition comprising the progranulin polypeptide can vary significantly depending on the patient condition, the disease state being treated, the route of administration of progranulin and tissue distribution, and the possibility of co-usage of other therapeutic treatments. The effective amount of a progranulin to be administered to the patient is based on body surface area, patient weight, physician assessment of patient condition, and the like. In one illustrative embodiment, an effective dose of a progranulin can range from about 1 ng/kg of patient body weight to about 1 mg/kg of patient body weight, more preferably from about 1 ng/kg of patient body weight to about 500 ng/kg of patient body weight, and most preferably from about 1 ng/kg of patient body weight to about 100 ng/kg of patient body weight.

In another illustrative embodiment, an effective dose of the progranulin polypeptide can range from about 1 pg/kg of patient body weight to about 1 mg/kg of patient body weight. In various illustrative embodiments, an effective dose can range from about 1 pg/kg of patient body weight to about 500 ng/kg of patient body weight, from about 500 pg/kg of patient body weight to about 500 ng/kg of patient body weight, from about 1 ng/kg of patient body weight to about 500 ng/kg of patient body weight, from about 100 ng/kg of patient body weight to about 500 ng/kg of patient body weight, and from about 1 ng/kg of patient body weight to about 100 ng/kg of patient body weight.

In another illustrative embodiment, an effective dose of the progranulin polypeptide can range from about 1 µg/kg of patient body weight to about 1 mg/kg of patient body weight. In various illustrative embodiments, an effective dose can range from about 1 µg/kg of patient body weight to about 500 µg/kg of patient body weight, from about 500 ng/kg of patient body weight to about 500 µg/kg of patient body weight, from about 1 µg/kg of patient body weight to about 500 µg/kg of patient body weight, from about 0.1 µg/kg of patient body weight to about 5 µg/kg of patient body weight, from about 0.1 µg/kg of patient body weight to about 10 µg/kg of patient body weight, and from about 0.1 µg/kg of patient body weight to about 100 µg/kg of patient body weight.

The composition comprising a progranulin is preferably administered to the patient parenterally, e.g., intradermally, subcutaneously, intramuscularly, intraperitoneally, intravenously, intraventricularly, intrathecally, intracerebrally or intracordally (spinal). Alternatively, the progranulin composition may be administered to the patient by other medically useful processes, and any effective dose and suitable therapeutic dosage form, including prolonged or sustained release dosage forms, can be used. Administration can be by injection. The composition comprising progranulin can also be delivered using a slow pump.

Examples of parenteral dosage forms include aqueous solutions of the active agent, in an isotonic saline, 5% glucose or other well-known pharmaceutically acceptable liquid carriers such as liquid alcohols, glycols, esters, and amides. The parenteral dosage form in accordance with this invention can be in the form of a reconstitutable lyophilizate comprising a dose of a composition comprising progranulin. In one aspect of the present embodiment, any of a number of prolonged or sustained release dosage forms known in the art can be administered such as, for example, the biodegradable carbohydrate matrices described in U.S. Patent Nos. 4,713,249; 5,266,333; and 5,417,982.

In an illustrative embodiment pharmaceutical formulations for general use with progranulins for parenteral administration comprising: a) a pharmaceutically active amount of the progranulin; b) a pharmaceutically acceptable pH buffering agent to provide a pH in the range of about pH 4.5 to about pH 9; c) an ionic strength modifying agent in the concentration range of about 0 to about 250 millimolar; and d) water soluble viscosity modifying agent in the concentration range of about 0.5% to about 7% total formula weight are described or any combinations of a), b), c) and d).

In various illustrative embodiments, the pH buffering agents for use in the compositions and methods herein described are those agents known to the skilled artisan and include, for example, acetate, borate, carbonate, citrate, and phosphate buffers, as well as hydrochloric acid, sodium hydroxide, magnesium oxide, monopotassium phosphate, bicarbonate, ammonia, carbonic acid, hydrochloric acid, sodium citrate, citric acid, acetic acid, disodium hydrogen phosphate, borax, boric acid, sodium hydroxide, diethyl barbituric acid, and proteins, as well as various biological buffers, for example, TAPS, Bicine, Tris, Tricine, HEPES, TES, MOPS, PIPES, Cacodylate, MES.

In another illustrative embodiment, the ionic strength modulating agents include those agents known in the art, for example, glycerin, propylene glycol, mannitol, glucose, dextrose, sorbitol, sodium chloride, potassium chloride, and other electrolytes.

Useful viscosity modulating agents include but are not limited to, ionic and non-ionic water soluble polymers; crosslinked acrylic acid polymers such as the "carbomer" family of polymers, e.g., carboxypolyalkylenes that may be obtained commercially under the Carbopol® trademark; hydrophilic polymers such as polyethylene oxides, polyoxyethylene-polyoxypropylene copolymers, and polyvinylalcohol; cellulosic polymers and cellulosic polymer derivatives such as hydroxypropyl cellulose, hydroxyethyl cellulose, hydroxypropyl methylcellulose, hydroxypropyl methylcellulose phthalate, methyl cellulose, carboxymethyl cellulose, and etherified cellulose; gums such as tragacanth and xanthan gum; sodium alginate; gelatin, hyaluronic acid and salts thereof, chitosans, gellans or any combination thereof. It is preferred that non-acidic viscosity enhancing agents, such as a neutral or basic agent be employed in order to facilitate achieving the desired pH of the formulation. If a uniform gel is desired, dispersing agents such as alcohol, sorbitol or glycerin can be added, or the gelling agent can be dispersed by trituration, mechanical mixing, or stirring, or combinations thereof. In one embodiment, the viscosity enhancing agent can also provide the base, discussed above. In one preferred embodiment, the viscosity modulating agent is cellulose that has been modified such as by etherification or esterification.

In various illustrative embodiments, progranulin compositions are provided that may comprise all or portions of progranulin polypeptides, alone or in combination with at least one other agent, such as an excipient and/or a stabilizing compound and/or a solubilizing agent, and may be administered in any sterile, biocompatible pharmaceutical carrier, including, but not limited to, saline, buffered saline, dextrose, glucose, and water. Suitable excipients are carbohydrate or protein fillers such as sugars, including lactose, sucrose, mannitol, or sorbitol; starch from corn, wheat, rice, potato, etc; cellulose such as methyl cellulose, hydroxypropylmethyl-cellulose, or sodium carboxymethylcellulose; and gums including arabic and tragacanth; and proteins such as gelatin and collagen. Suitable disintegrating or solubilizing agents include agar, alginic acid or a salt thereof such as sodium alginate.

In illustrative embodiments, progranulin polypeptides can be administered to a patient alone, or in combination with other agents, drugs or hormones or in pharmaceutical compositions where it is mixed with excipient(s) or other pharmaceutically acceptable carriers. In one embodiment, the pharmaceutically acceptable carrier is pharmaceutically inert. In another embodiment, progranulin polypeptides may be administered alone to a patient suffering from a neurological disease.

Any effective regimen for administering the composition comprising progranulin can be used. For example, the composition comprising progranulin can be administered as a single dose, or the composition comprising progranulin can be divided and administered as a multiple-dose daily regimen. Further, a staggered regimen, for example, one to three days per week can be used as an alternative to daily treatment, and for the purposes of this invention such intermittent or staggered daily regimen is considered to be equivalent to every day treatment and within the scope of this invention. In one embodiment, the patient is treated with multiple injections of the composition comprising progranulin to decrease neuronal cell death. In another embodiment, the patient is injected multiple times (e.g., about 2 up to about 50 times) with the composition comprising progranulin, for example, at 12-72 hour intervals or at 48-72 hour intervals. Additional injections of the composition comprising progranulin can be administered to the patient at an interval of days or months after the initial injections(s) and the additional injections prevent recurrence of disease. Alternatively, the initial injection(s) of the composition comprising progranulin may prevent recurrence of disease.

In another illustrative embodiment, patients with a neurodegenerative disease can be treated by administering to the patient a composition comprising an effector (e.g., a DNA encoding a therapeutic molecule, such as DNA's encoding progranulin or portions of progranulin), or combinations of effectors, that modifies progranulin expression, wherein treatment of the patient with the composition comprising the effector that modifies progranulin expression reduces the symptoms of the neurological disease in the patient.

In yet another embodiment, a pharmaceutical composition is provided. The pharmaceutical composition comprises therapeutically effective amounts of an effector that modifies progranulin expression, and a pharmaceutically acceptable carrier, wherein the therapeutically effective amounts comprise amounts capable of reducing or preventing the symptoms of a neurodegenerative disease.

In another embodiment, a method is provided for reducing neuronal cell death in a patient. The method comprises the steps of administering to a patient with a neurodegenerative disease a therapeutically effective amount of an effector that modifies progranulin expression, wherein the amount of effector is effective to increase neuronal cell survival or proliferation in the patient. In another illustrative embodiment, the amount of effector is effective to increase the expression of progranulin in neurons. In further illustrative embodiments, the amount of effector is effective to decrease the expression of progranulin in non-neuronal cells.

In another illustrative embodiment, patients with a neurodegenerative disease mediated by limit to DNA's encoding progranulin on portions of progranulin an environmental insult can be treated by administering to the patient a composition comprising an effector (e.g., a DNA encoding a therapeutic molecule), or combinations of effectors, that modifies progranulin expression, wherein treatment of the patient with the composition comprising the effector that modifies progranulin expression reduces the symptoms of the neurological disease in the patient.

In yet another embodiment, a pharmaceutical composition is provided. The pharmaceutical composition comprises therapeutically effective amounts of an effector that modifies progranulin expression, and a pharmaceutically acceptable carrier, wherein the therapeutically effective amounts comprise amounts capable of reducing or preventing the symptoms of a neurodegenerative disease mediated by an environmental insult to a patient.

In another embodiment, a method is provided for reducing neuronal cell death in a patient. The method comprises the steps of administering to a patient with a neurodegenerative disease mediated by an environmental insult a therapeutically effective amount of an effector that modifies progranulin expression, wherein the amount of effector is effective to increase neuronal cell survival or proliferation in the patient. In another illustrative embodiment, the amount of effector is effective to increase the expression of progranulin in neurons. In further illustrative embodiments, the amount of effector is effective to decrease the expression of progranulin in non-neuronal cells.

As used herein, "an effector that modifies progranulin expression" means a nucleic acid (e.g. a DNA, a cDNA, or an mRNA) that increases progranulin expression in target cells. As used herein "target cells" comprise neuronal cells. The unitary daily dosage of the composition comprising the effector that modifies progranulin expression can vary significantly depending on the patient condition, the disease state being treated, the molecular weight of the effector, its route of administration and tissue distribution, and the possibility of co-usage of other therapeutic treatments. The effective amount to be administered to the patient is based on body surface area, patient weight, and physician assessment of patient condition. In one illustrative embodiment, an effective dose of the effector can range from about 1 ng/kg of patient body weight to about 1 mg/kg of patient body weight, more preferably from about 1 ng/kg of patient body weight to about 500 ng/kg of patient body weight, and most preferably from about 1 ng/kg of patient body weight to about 100 ng/kg of patient body weight.

In another illustrative embodiment, an effective dose of the effector can range from about 1 pg/kg of patient body weight to about 1 mg/kg of patient body weight. In various illustrative embodiments, an effective dose can range from about 1 pg/kg of patient body weight to about 500 ng/kg of patient body weight, from about 500 pg/kg of patient body weight to about 500 ng/kg of patient body weight, from about 1 ng/kg of patient body weight to about 500 ng/kg of patient body weight, from about 100 ng/kg of patient body weight to about 500 ng/kg of patient body weight, and from about 1 ng/kg of patient body weight to about 100 ng/kg of patient body weight.

In another illustrative embodiment, an effective dose of the effector can range from about 1 million effector molecules per 70 kg patient body to about 1 billion effector molecules per 70 kg patient body. In various illustrative embodiments, an effective dose can range from about 1 million effector molecules per 70 kg patient body to about 500 million effector molecules per 70 kg patient body, from about 200,000 effector molecules per 70 kg patient body to about 200 million effector molecules per 70 kg patient body, from about 1 million effector molecules per 70 kg patient body to about 200 million effector molecules per 70 kg patient body.

The composition comprising the effector that modifies progranulin expression is preferably administered to the patient parenterally, e.g., intradermally, subcutaneously, intramuscularly, intraperitoneally, intravenously, intraventricularly, intrathecally, intracerebrally or intracordally (spinal). Alternatively, the composition comprising the effector that modifies progranulin expression may be administered to the patient by other medically useful processes, and any effective dose and suitable therapeutic dosage form, including prolonged release dosage forms, can be used. Administration can be accomplished by injection.

The composition comprising the effector that modifies progranulin expression is preferably injected parenterally and such injections can be intradermal injections, intraperitoneal injections, subcutaneous injections, intramuscular injections, intravenous injections, intraventricular injections, intrathecal injections, intracerebral injections or intracordal injections (spinal). The composition comprising the effector that modifies progranulin expression can also be delivered using a slow pump. Additionally, suitable routes may, for example, include oral or transmucosal administration. Therapeutic administration of an effector that modifies progranulin expression intracellularly can also be accomplished as described below. Examples of parenteral dosage forms include aqueous solutions of the active agent, in an isotonic saline, 5% glucose or other well-known pharmaceutically acceptable liquid carriers such as liquid alcohols, glycols, esters, and amides. The parenteral dosage form in accordance with this invention can be in the form of a reconstitutable lyophilizate comprising a dose of a composition comprising an effector that modifies progranulin expression. In one aspect of the present embodiment, any of a number of prolonged release dosage forms known in the art can be administered such as, for example, the biodegradable carbohydrate matrices described in U.S. Patent Nos. 4,713,249; 5,266,333; and 5,417,982.

Any effective regimen for administering the composition comprising the effector that modifies progranulin expression can be used. For example, the composition comprising the effector that modifies progranulin expression can be administered as a single dose, or the composition comprising the effector that modifies progranulin expression can be administered in multiple doses. Further, a staggered regimen, for example, one to three days per week can be used as an alternative to daily treatment, and for the purposes of this invention such intermittent or staggered daily regimen is considered to be equivalent to every day treatment and within the scope of this invention. In one embodiment, the patient is treated with one or more injections of the composition comprising the effector that modifies progranulin expression. In another embodiment, the patient is injected multiple times (e.g., about 2 up to about 50 times) with the composition comprising the effector that modifies progranulin expression, for example, at 12-72 hour intervals or at 48-72 hour intervals. Additional injections of the composition comprising the effector that modifies progranulin expression can be administered to the patient at an interval of days or months after the initial injections(s) and the additional injections prevent recurrence of disease. Alternatively, the initial one or more injection(s) of the composition comprising the effector that modifies progranulin expression may prevent recurrence of disease.

In various illustrative embodiments, the presently described compositions comprise an isolated and purified nucleic acid sequence encoding the progranulin gene or a portion thereof. Methods of purifying nucleic acids are well-known to those skilled in the art. In one embodiment, the sequence is operatively linked to regulatory sequences directing expression of the progranulin gene. In further embodiments, the sequence is operably linked to a heterologous promoter. In still further embodiments, the sequence is contained within a vector. In some embodiments, the vector is within a host cell (e.g., a neuronal cell).

As used herein, the term "vector" is used in reference to nucleic acid molecules that transfer DNA or mRNA segment(s) to cells in the patient. The vector contains the nucleic acid sequence and appropriate nucleic acid sequences necessary for the expression of the operably linked nucleic acid coding sequence in the patient. A vector is capable of expressing a nucleic acid molecule inserted into the vector and, of producing a polypeptide or protein. Nucleic acid sequences necessary for expression usually include a promoter, an operator (optional), and a ribosome binding site, often along with other sequences such as enhancers, and termination and polyadenylation signals.

If a cell is used for delivery of the nucleic acid, the nucleic acid may be introduced into the cell by transducing, transfecting, microinjecting, or electroporating, the cell with the nucleic acid. A delivery cell may be transformed, transduced, or transfected (e.g., by calcium phosphate-DNA co-precipitation, DEAE-dextran-mediated transfection, polybrene-mediated transfection, electroporation, microinjection, liposome fusion, lipofection, protoplast fusion, retroviral infection, biolistics, etc.) by exogenous or heterologous nucleic acids when such nucleic acids have been introduced inside the cell. Transforming DNA, for example, may or may not be integrated (covalently linked) with chromosomal DNA making up the genome of the delivery cell. In mammalian cells for example, transforming DNA may be maintained on an episomal element, such as a plasmid. In a eukaryotic cell, a stably transformed cell is one in which the transforming DNA has become integrated into a chromosome so that it is inherited by daughter cells through chromosome replication.

As used herein, the effector that modifies progranulin expression can comprise a "progranulin nucleic acid" and the progranulin nucleic acid comprises a complete progranulin coding sequence or a homologous sequence as described herein.

In another illustrative embodiment, a progranulin nucleic acid can be incorporated into a vector and administered to a patient by any protocol known in the art such as those described in U.S. Patent Nos. 6,333,194, 7,105,342 and 7,112,668. In illustrative embodiments, progranulin nucleic acid, can be introduced either *in vitro* into a cell extracted from an organ of the patient wherein the modified cell then being reintroduced into the body, or directly *in vivo* into the appropriate tissue or using a targeted vector-progranulin nucleic acid construct. In various illustrative embodiments, the progranulin nucleic acid can be introduced into a cell or an organ using, for example, a viral vector, a retroviral vector, or non-viral methods, such as transfection, injection of naked DNA, electroporation, sonoporation, a "gene gun" (e.g., by shooting DNA coated gold particles into cells using high pressure gas), synthetic oligomers, lipoplexes, polyplexes, virosomes, or dendrimers.

In one embodiment where cells or organs are treated, the progranulin nucleic acid can be introduced into a cell or organ using a viral vector. The viral vector can be any viral vector known in the art. For example, the viral vector can be an adenovirus vector, a lentivirus vector, a retrovirus vector, an adeno-associated virus vector, a herpesvirus vector, a modified herpesvirus vector, and the like. In another illustrative embodiment where cells are transfected, the progranulin nucleic acid can be introduced into a cell by direct DNA transfection (lipofection, calcium phosphate transfection, DEAE-dextran, electroporation, and the like).

In various illustrative embodiments, the progranulin nucleic acid can be, for example, a DNA molecule, an RNA molecule, a cDNA molecule, or an expression construct comprising a progranulin nucleic acid.

The progranulin nucleic acids described herein can be prepared or isolated by any conventional means typically used to prepare or isolate nucleic acids and include the nucleic acids of SEQ ID. No. (1) and (13). For example, DNA and RNA molecules can be chemically synthesized using commercially available reagents and synthesizers by methods that arc known in the art. The progranulin nucleic acids described herein can be purified by any conventional means typically used in the art to purify nucleic acids. For example, the progranulin nucleic acids can be purified using electrophoretic methods and nucleic acid purification kits known in the art (e.g. Quigen kits). Progranulin nucleic acids suitable for delivery using a viral vector or for introduction into a cell by direct DNA transfection can also be prepared using any of the recombinant methods known in the art.

Nucleic acids having modified internucleoside linkages can also be used in the methods and compositions herein described. Nucleic acids containing modified internucleoside linkages can be synthesized using reagents and methods that are known in the art, for example, methods for synthesizing nucleic acids containing phosphonate, phosphorothioate, phosphorodithioate, phosphoramidate methoxyethyl phosphoramidate, formacetal, thioformacetal, diisopropylsilyl, acetamidate, carbamate, dimethylene-sulfide (-CH.sub.2 --S--CH.sub.2 --), dimethylene-sulfoxide (--CH.sub.2 --SO--CH.sub.2 --), dimethylene-sulfone (--CH.sub.2 --SO.sub.2 --CH.sub.2 --), 2'-O-alkyl, and 2'-deoxy-2'-fluorophosphorothioate internucleoside linkages.

Modified progranulin sequences, i.e. sequences that differ from the sequence encoding native progranulin, are also encompassed by the invention, so long as the modified sequence still encodes a protein that exhibits the biological activity of the native progranulin at a greater or lesser level of activity. These modified progranulin sequences include modifications caused by point mutations, modifications due to the degeneracy of the genetic code or naturally occurring allelic variants, and further modifications that are introduced by genetic engineering, to produce recombinant progranulin nucleic acids.

Progranulin nucleic acids include nucleic acids with 95% homology to SEQ ID Nos. 1 and 13 or to nucleic acids which hybridize under highly stringent conditions to the complement of the DNA coding sequence for a progranulin SEQ ID Nos. 1 or 13. As used herein, the term "hybridization" is used in reference to the pairing of complementary nucleic acids. Hybridization and the strength of hybridization (e.g., the strength of the association between the nucleic acids) is impacted by such factors as the degree of complementary between the nucleic acids, stringency of the conditions involved, the Tₘ (melting temperature) of the formed hybrid, and the G:C ratio within the nucleic acids. As used herein the term " stringency " is used in reference to the conditions of temperature, ionic strength, and the presence of other compounds such as organic solvents, under which nucleic acid hybridizations are conducted.

In an illustrative example, "highly stringent conditions" can mean hybridization at 65 °C in 5X SSPE and 50% formamide, and washing at 65 °C in 0.5X SSPE. In another illustrative example, "highly stringent conditions" can mean hybridization at 55°C in a hybridization buffer consisting of 50% formamide (vol/vol); 10% dextran sulfate; 1 x Denhardt's solution; 20 mM sodium phosphate, pH 6.5; 5 x SSC; and 200 µg of salmon sperm DNA per ml of hybridization buffer for 18 to 24 hours, and washing four times (5 min each time) with 2 x SSC; 1% SDS at room temperature and then washing for 15 min at 50-55°C with 0.1 x SSC. In another illustrative example Conditions for high stringency hybridization are described in Sambrook et al., "Molecular Cloning: A Laboratory Manual", 3rd Edition, Cold Spring Harbor Laboratory Press, (2001). In some illustrative aspects, hybridization occurs along the full-length of the nucleic acid.

In various embodiments of the methods and compositions described herein, the probes can be labeled, such as with fluorescent compounds, radioactive isotopes, antigens, biotin-avidin, colorimetric compounds, or other labeling agents known to those of skill in the art, to allow detection and quantification of amplified DNA, such as by Real-Time PCR. In illustrative embodiments, the labels may include 6-carboxyfluorescein (FAM™), TET™ (tetrachloro-6-carboxyfluorescein), JOE™ (2,7, -dimethoxy-4,5-dichloro-6-carboxyfluorescein), VIC™, HEX (hexachloro-6-carboxyfluorescein), TAMRA™ (6-carboxy-N,N,N',N'-tetramethylrhodamine), BHQ™, SYBR® Green, Alexa 350, Alexa 430, AMCA, BODIPY 630/650, BODIPY 650/665, BODIPY-FL, BODIPY-R6G, BODIPY-TMR, BODIPY-TRX, Cascade Blue, Cy3, Cy5,6-FAM, Fluorescein, Oregon Green 488, Oregon Green 500, Oregon Green 514, Pacific Blue, REG, Rhodamine Green, Rhodamine Red, ROX, and/or Texas Red.

Detection of highly stringent hybridization in the context of the present invention indicates strong structural similarity or structural homology (e.g., nucleotide structure, base composition, arrangement or order) to, e.g., the nucleic acids provided herein.

Also included are nucleic acid molecules having about 80%, about 85%, about 90%, about 95%, 96%, 97%, 98%, and 99% homology to the DNA coding sequence for a progranulin SEQ ID No. 1 or 13. As used herein, the percent homology between two sequences is equivalent to the percent identity between the sequences. Determination of percent identity or homology between sequences can be done, for example, by using the GAP program (Genetics Computer Group, software; now available via Accelrys on http://www.accelrys.com), and alignments can be done using, for example, the ClustalW algorithm (VNTI software, InforMax Inc.). A sequence database can be searched using the nucleic acid sequence of interest. Algorithms for database searching are typically based on the BLAST software (Altschul et al., 1990). In some embodiments, the percent homology oridentity can be determined along the full-length of the nucleic acid.

As used herein, the term "complementary" refers to the ability of purine and pyrimidine nucleotide sequences to associate through hydrogen bonding to form double-stranded nucleic acid molecules. Guanine and cytosine, adenine and thymine, and adenine and uracil are complementary and can associate through hydrogen bonding resulting in the formation of double-stranded nucleic acid molecules when two nucleic acid molecules have "complementary" sequences. The complementary sequences can be DNA or RNA sequences. The complementary DNA or RNA sequences are referred to as a "complement." Complementary may be "partial," in which only some of the nucleic acids' bases are matched according to the base pairing rules. Or, there may be "complete" or "total" complementary between the nucleic acids.

In illustrative embodiments, the neurodegenerative disease is mediated by an environmental insult to the patient. As used herein, a neurodegenerative disease mediated by an environmental insult to the patient means a disease that is caused by an environmental insult and is not caused by a heritable mutation of the progranulin gene that modifies progranulin expression. A heritable mutation is a permanent mutation in a patient's DNA that may be transmitted to the patient's offspring. These illustrative embodiments are however not meant to exclude the influence of allelic variants of modifier genes, that are, for example, involved in the metabolism of the neurotoxin, that render an individual more or less sensitive to neurodegenerative disease development. As used herein these modifier genes can modify the course of disease development.

The neurodegenerative disease mediated by environmental insult to the patient may be a sporadic disease linked to environmental factors that cause neuronal cell death directly or indirectly by modifying gene expression. In various other illustrative embodiments, the environmental insult is derived from the patient's diet or is the result of endogenous synthesis, or both. In one illustrative embodiment, the environmental insult causes synthesis of a compound that causes a detrimental effect *in vivo.* The neuronal cell death may occur by any variety of means including, but not limited to, excitotoxicity or oxidative stress. For example, various means by which environmental toxins lead to neuronal cell death are described in U.S. Patent Application Publication No. 2006-0252705.

In another illustrative embodiment, the neurodegenerative disease state is mediated by an excitotoxin. Excitotoxins are a class of substances that damage neurons through overactivation of receptors, for example, receptors for the excitatory neurotransmitter glutamate, leading to neuronal cell death. Examples of excitotoxins include excitatory amino acids, which can produce lesions in the central nervous system. Additional examples of excitotoxins include, but are not limited to, sterol glucoside, including beta-sitosterol-beta-D-glucoside and cholesterol glucoside, methionine sulfoximine, and other substances known in the art to induce neuro-excitotoxic reactions in a patient. In one illustrative embodiment, the excitotoxin is a sterol glycoside. In further illustrative embodiments, the sterol glycoside is selected from the group consisting of beta-sitosterol-beta-D-glucoside and cholesterol glucoside, or analogs or derivatives thereof.

In one illustrative embodiment, the neurodegenerative disease is selected from the group consisting of Parkinson's disease, Alzheimer's disease, and ALS. Neurological diseases, including Alzheimer's disease, Parkinson's disease, and ALS, generally result in behavioral deficits that can be observed clinically. These diseases target populations of neurons leading to neuropathological and behavioral symptoms. Alzheimer's disease involves the death of neurons of various regions of the cerebral cortex and the hippocampus and results in the loss of cognitive functions such as memory and learning. Parkinson's disease results in degeneration of portions of the nigral-striatal system. Initial stages involve the loss of terminal projections of dopamine-containing neurons from the substantia nigra. In turn, the neuron cell bodies in the substantia nigra die, impacting motor control and leading to tremor and gait disturbances.

An example of a motor neuron disease is amyotrophic lateral sclerosis (ALS). ALS primarily involves the loss of spinal and cortical motor neurons, leading to increasing paralysis and eventually death. Early symptoms of ALS include but are not limited to, footdrop or weakness in a patient's legs, feet, or ankles, hand weakness or clumsiness, muscle cramps and twitching in the arms, shoulders, and tongue. ALS generally affects chewing, swallowing, speaking, and breathing, and eventually leads to paralysis of the muscles required to perform these functions. A review of various neurological diseases is set forth in Shaw et al., Neuroscience and Biobehavioral Reviews, 27: 493 (2003). The method and compositions of the present invention can be used for both human clinical medicine and veterinary medicine applications. The methods and compositions described herein may be used alone, or in combination with other methods or compositions.

In another illustrative embodiment, patients with a neurodegenerative disease mediated by an environmental insult can be treated by administering to the patient a composition comprising an effector (e.g., a DNA encoding a therapeutic molecule), or combinations of effectors, that modifies progranulin expression, wherein treatment of the patient with the composition comprising the effector that modifies progranulin expression reduces the symptoms of the neurological disease in the patient. Any of the above embodiments using effectors that mediate progranulin expression are applicable to this embodiment.

In yet another embodiment, a pharmaceutical composition is provided. The pharmaceutical composition comprises therapeutically effective amounts of an effector that modifies progranulin expression, and a pharmaceutically acceptable carrier, wherein the therapeutically effective amounts comprise amounts capable of reducing or preventing the symptoms of a neurodegenerative disease mediated by an environmental insult to a patient. Any of the above embodiments using effectors that mediate progranulin expression are applicable to this embodiment.

In another embodiment, a method is provided for reducing neuronal cell death in a patient. The method comprises the steps of administering to a patient with a neurodegenerative disease mediated by an environmental insult a therapeutically effective amount of an effector that modifies progranulin expression, wherein the amount of effector is effective to increase neuronal cell survival or proliferation in the patient. In another illustrative embodiment, the amount of effector is effective to increase the expression of progranulin in neurons. In further illustrative embodiments, the amount of effector is effective to decrease the expression of progranulin in non-neuronal cells. Any of the above embodiments using effectors that mediate progranulin expression are applicable to this embodiment.

### EXAMPLE 1

### ANIMALS

*In vivo* experiments were conducted using CD-1 colony reared 5-7 month old male mice purchased from Charles River (Wilmington, MA).

### EXAMPLE 2

### MICROSCOPY

Microscopy and all photomicrographs from mouse sections were captured using a Motic B5 Professional Series 3.0 (Motic Instruments Inc., Richmond, Canada) camera and Zeiss Axiovert Epiflorescence 2000 microscope. Data were analyzed using Motic B5 Professional, Motic Images Advanced 3.0 and Zeiss Axiovert Zoom Axiovision 3.1 with AxioCam HRM.

### EXAMPLE 3

### STATISTICAL ANALYSIS

For behavioral and histological experiments, values for each mouse on the individual tasks were used to calculate means ± S.E.M. for each group. The means were compared using an unpaired, two-tailed t-test or a one-way ANOVA. *A post hoc* Tukey's test was to compare all means after ANOVA testing (GraphPad Prism, San Diego, CA).

### EXAMPLE 4

### PROGRESSIVE DECREASE IN LEG EXTENSION REFLEX FOLLOWING SYNTHETIC STERYL GLUCOSIDE EXPOSURE

Three month old male CD-1 mice were fed 100 mg/day of synthetic BSSG for 15 weeks, then permitted to age with a diet of normal lab chow. Leg extension was measured weekly from the initiation of the experiment. The results are shown in Figure 1. Testing was performed as described in Wilson et al., 2004 and Wilson et al., 2005. Non-linear regression analysis demonstrates a significant difference at * = p < 0.0001 (plotted from week 0-32) in the data for the control mice and the BSSG-fed mice. Even following the cessation of BSSG exposure, the decline in leg extension reflex in the BSSG-fed mice continued to progress for the duration of the experiment.

The leg extension reflex test was used as a measure of motor neuron dysfunction (Barneoud and Curet, 1999). This test was altered to discriminate more subtle behaviors, creating a scale from 0 to 4. This scaled test shows the progressive loss of function as the normal reflex usually deteriorates progressively to a tremor and then to total retraction. This scale allows the measure of progression in a continuous manner over time. A score of 0-4 is assigned based on the response shown by the mouse as follows:
4: Complete extension of both legs (normal).
3: Two legs extended with some tremors and/or punching in a leg.
2: One leg extended, 1 retracted, or tremors in both legs.
1: One leg retracted, tremors in other leg.
0: Both legs retracted.

### EXAMPLE 5

### PROGRESSIVE DECREASE OF OPEN FIELD MOTOR ACTIVITY FOLLOWING SYNTHETIC STERYL GLUCOSIDE EXPOSURE

The same mice exposed to BSSG in Example 4 were used to analyze open field motor activity. * = p < 0.05 (Student T-test). The results are shown in Figure 2. BSSG-fed mice showed significantly decreased movement as measured by grid crossing at week 28 compared to controls. Following the cessation of BSSG exposure, the decline in open field motor activity was observed to progress with time.

For the open field motor activity analysis, mice were placed in a round open field (2 m diameter) for 5 minutes and movements were recorded using a video camera to measure emotionality and exploration (spontaneous motor activity) (Karl et al., 2003). Videos were replayed on a TV and a circular grid was overlaid on the TV screen. Grid crossings were recorded.

### EXAMPLE 6

### PROGRESSIVE LOSS OF MOTOR NEURONS FOLLOWING 10 WEEKS EXPOSURE TO SYNTHETIC STERYL GLUCOSIDE

Six month old male CD-1 mice were fed varying doses of synthetic BSSG for 10 weeks, then allowed to age for 1 month with a diet of normal chow prior to sacrifice (see Figure 3, panels A-C). Motor neurons were quantified in lumbar cord, by Nissl staining and choline acetyltransferase (ChAT) immunohistochemistry (IHC), as shown in Figure 3A. Motor neurons were quantified in motor cortex where neurons were detected by IHC against CTIP2 (highly expressed in corticospinal motor neurons), as shown in Figure 3C. Activated caspase-3 was detected in lumbar spinal cord (ventral horn) by IHC, as shown in Figure 3B. Motor neurons in the lumbar spinal cord of animals permitted to age for 5 months following the cessation of BSSG were quantified after Nissl staining, as shown in Figure 3D (* P < 0.01, ANOVA). Progressive loss of motor neurons is seen when mice were permitted to age in the absence of further BSSG exposure.

Lumbar spinal cord sections were stained with cresyl violet and ventral horn motor neurons were counted under a 40 X objective lens. All motor neurons in the field of view were included in the results. Multiple sections (N=6) from each mouse were used. Counts were conducted on spinal cord sections that were at least 150 µm apart (in the rostral-caudal plane) ensuring that no motor neuron was counted twice. In addition, counts included all apparent motor neurons including motor neurons that may have appeared atrophic or damaged.

Active caspase-3 (Promega, Madison, WI) labeling was performed as follows. Active caspase-3 levels were identified by immunohistochemistry based on previous work (Schulz ct al., 2003; Wilson et al., 2005). Briefly, slide mounted sections were incubated in blocking solution for 2 hours and then with the primary antibody (Casp-3 1:250, raised in rabbit) overnight at room temperature. Sections were rinsed and incubated in fluorescent secondary antibodies (anti-rabbit IgG 1:200, Vector laboratories Inc., Burlingame, CA) for 2 hours. Sections were visualized using fluorescence microscopy. Mounting medium with DAPI (Vector Laboratories, Inc., Burlingame, CA) was used to counterstain all nuclei.

After the behavioral testing, all animals were anaesthetized with halothane and perfused by cardiac puncture perfusion with chilled PBS and 4% paraformadehyde (PFA). Brain and spinal cord samples were then immersed in 4% PFA, for 2 days, cryoprotected in 20% sucrose 0.5% sodium azide solution for 1 day, and frozen until sectioning for immunohistochemistry. This assay can be performed by any immunohistochemical staining procedure known in the art.

### EXAMPLE 7

### IN SITU HYBRIDIZATION TO DETECT PROGRANULIN EXPRESSION IN THE BRAINSTEM OF A NORMAL MOUSE

ISH using progranulin specific antisense riboprobes was performed on paraffin embedded normal adult mouse brain cut in sagittal section. High levels of progranulin expression were detected in large cells having a neuronal morphology, as shown in Figure 4A at magnification 10 X and Figure 5A at magnification 40 X. Figure 4B and Figure 5B show control sections at magnification 10 X and magnification 40 X, respectively, incubated with sense probes.

### EXAMPLE 8

### IN SITU HYBRIDIZATION TO DETECT PROGRANULIN EXPRESSION IN ANTERIOR HORN CELLS OF THE CERVICAL SPINAL CORD OF A NORMAL MOUSE

ISH using progranulin specific antisense riboprobes was performed on frozen sections of normal adult mouse spinal cord cut in trans-section. High levels of progranulin expression were detected in the anterior horn motor neurons, as shown in Figure 6A versus a control section shown in Figure 6B, incubated with a sense probe.

### EXAMPLE 9

### DECREASED PROGRANULIN EXPRESSION IN THE MOTOR CORTEX OF WASHED CYCAD FLOUR FED MICE

Adult male CD-1 mice were fed a diet of either normal chow or chow containing 1 gram per day of washed CYCAD flour. Following 10 weeks of feeding, the mice were killed and ISH using progranulin specific riboprobes was performed on frozen sections of motor cortex. High levels of progranulin expression were detected in layers 4-5 of the cortex of the control mouse using an antisense probe (Figure 7 A). In contrast, using an antisense probe, the cortex of the CYCAD-fed mouse exhibited blunted progranulin expression as shown in Figure 7B. There was a decrease in progranulin expression on a per cell basis in the tissue from the CYCAD-fed mouse. There was no evidence of gross disruption of the architecture of the cortex following CYCAD treatment. Figure 7C shows a control section incubated with a sense probe (magnification 10X). In Figure 8 A-C the same sections shown in Figure 7 are depicted at a higher magnification (magnification 40X).

### EXAMPLE 10

### EXPOSURE TO SYNTHETIC BSSG RESULTS IN DECREASED PROGRANULIN EXPRESSION IN CERVICAL SPINAL CORD

Adult male CD-1 mice were fed a diet of either normal chow or chow containing 1000 µg per day of synthetic BSSG. Following 10 weeks of feeding with BSSG-containing chow and a further month with normal lab chow, mice were killed and ISH using progranulin specific antisense riboprobes was performed on frozen sections of cervical spinal cord cut in trans-section. High levels of progranulin expression were detected in the anterior horn cells of the control mouse as shown by the section in Figure 9A. In the BSSG-treated mouse (section shown in Figure 9 B), progranulin expression was both decreased in intensity and was observed in fewer cells of the anterior horn. A negative control section is shown in Figure 9C, incubated with a sense probe.

### EXAMPLE 11

### INCREASING BSSG EXPOSURE RESULTS IN MORE PRONOUNCED NEUROPATHOLOGY AND LOSS OF PROGRANULIN EXPRESSION IN CERVICAL SPINAL CORD

Adult male CD-1 mice were fed a diet of chow containing 10, 100, or 1000 µg per day of synthetic BSSG. Following 10 weeks of feeding with BSSG- containing chow and a further month with normal lab chow, mice were killed and ISH using progranulin specific riboprobes was performed on frozen sections of cervical spinal cord cut in trans-section. A more pronounced neuropathology was associated with increased exposure to BSSG. A progressive loss of cells expressing progranulin and exhibiting a motor neuron morphology are apparent with increased exposure to BSSG. Additionally, there is a more pronounced loss of progranulin expression per cell as BSSG exposure increases. Mice assessed in Figures 9 and 10 are the same mice as analyzed in Example 3, where a dose dependent loss of motor neuron health was observed in lumbar spinal cord (see Figure 3, Panels A, B, and D).

### EXAMPLE 12

### KNOCKDOWN OF PROGRANULIN EXPRESSION IN ZEBRAFISH LEADS TO LOSS OF VENTRAL HORN CELLS

### Zebrafish strains and husbandry

Wild type zebrafish (*zdr* strain) were purchased from Aquatica Tropicals Inc. (Plant City FL) and maintained on a 14 h/10 h light/dark cycle at 28.5° C in a laboratory aquarium (Allantown Aquaneering, Allantown, NJ). Fish were fed twice daily, and bred as described elsewhere (Mullins et al, 1994). Embryos for developmental studies were collected from tanks and staged according to conventional criteria (Kimmel et al, 1995) and by hours post-fertilization (hpf).

### Embryo microinjections

Morpholinos (Gene Tools LLC, Philomath OR) were resuspended in 100uL sterile water at a concentration of 25ng/*u*L. The injection solutions consisted of 1-15ng/nL morpholino (MO) diluted in Danieu buffer (58 mM NaCl, 0.7 mM KCl, 0.4 mM MgSO₄, 0.6 mM Ca(NO₃)₂, 5.0 mM HEPES; pH 7.6), and 0.05% phenol red was included as visual tracer (Nasevicius et al, 2000). Zebrafish embryos at 1- and 2-cell stages were injected with 1-15ng MO/embryo. Morpholinos designed to target the 5'UTR of zebrafish pgrn-a and controls are as follows:
MO2-UTR, 5'-GAGCAGGTGGATTTGTGAACAGCGG-3'
MO2-mismatch, 5'-GAACACGTGGATTTCTGAAGAGAGG-3'
Scramble, 5'-CCTCTTACCTCAGTTACAATTTATA-3'.

Concentrations of 15ng/embryo (7.5ng/nl) was considered the upper limit as the scramble control produces a consistent minority of non-specific developmental defects at this concentration. Embryos were allowed to develop at 28.5° C until harvested for molecular analysis at varying developmental stages.

### Whole-mount immunofluorescence

For Zn8 immunostaining embryos were fixed using 4% paraformaldehyde (PFA) in phosphate buffered saline (PBS) for 2 hours at room temperature and then stored in 100% methanol at -20° C. Embryos were rehydrated with PBST (100mM Na₂HPO₄, 20mM KH₂PO₄, 137mM NaCl, 27mM KCl, 0.1% Tween-20, pH 7.4) and permeabilized by digesting with 10µg/ml proteinase K for 20 minutes followed by post-fixed in 4%PFA/PBS for 20 minutes. After several PBST washes embryos were blocked in PBST containing 5% calf serum. After three hours the primary antibody monoclonal anti-Zn8 (ZIRC, Eugene OR) was added at a 1:1000 dilution and incubated overnight at 4° C. After extensive washing in PBST embryos were incubated with Alexa488 conjugated anti-mouse (Invitrogen) at 1:200 for 2 hours in PBST with 5% calf serum. Fluorescence was visualized with a Leica MZ FLIII stereomicrosope equipped with a GFP filter.

As shown in Fig. 11, a knockdown of progranulin expression in zebrafish leads to the morphological manifestations of craniofacial dysmorphogenesis, pericardial edema, and visceral gut distention. Additionally, although not visible in the photograph, a loss of motor neurons was observed.

### EXAMPLE 13

### NSC34 CELL CULTURE

The NSC34 cell line was maintained in DMEM with 10% fetal bovine serum unless otherwise stated [see Cashman et al., Dev Dyn. 194:209-21 (1992)J. For stable transfections NSC34 cells were transfected with human progranulin (pcDNA-Pgrn) or empty vector (pcDNA) using Lipofectamine (Invitrogen) and selected with G418 for one month according to manufacturer's instructions. Serum deprivation assays were carried out in 6-well plates using 200,000 cells/well and cultured in 4ml of RPMI (with glutamine) for 3, 6, 9, 12 and 15 days without the addition or exchange of fresh medium. For each time point the average cell number was determined over 6 visual fields per well at 10X magnification using an Olympus phase-contrast microscope (Figure 14, Panel A).

For hypoxia assays the cells were plated at a density of 50,000/well in 24-well plates, starved for 24 hours in RPMI without serum followed by the addition of fresh serum free RPMI or DMEM containing 5% serum and maintained in a hypoxia chamber containing 1% O₂, 5% CO₂, balance N₂ for 72 hours. Cells were maintained in the hypoxic environment for 3 days, trypsinized and counted using a hemocytometer (Figure 15).

For long term cultures NSC34 cells were plated at a density of 200,000/well in 6-well plates and maintained in serum free RPMI medium. Fresh medium was provided every 10 days and 10X magnification photos taken at 20 and 57 days using an Olympus phase-contrast microscope (Figure 16).

### EXAMPLE 14

### NSC34 CELL IMMUNOFLUORESCENCE

The NSC34 cell line, together with stable transfectants were cultured on glass coverslips in DMEM with 10% fetal bovine serum. Cells were fixed in 4% PFA, rinsed twice with PBST, and incubated with permeabilization buffer (PBST with 0.2% Triton X-100) for 20 minutes. After being washed three times with PBST, the cultures were post-fixed for 10 minutes with 4% PFA, followed by extensive washing. Fixed cells were incubated in PBST with 0.5% (w/v) membrane blocking reagent (GE Healthcare) for one hour followed by the addition of sheep anti-mouse progranulin, (1:500 dilution, R&D Systems).

Incubation with the primary antibody continued overnight at 4°C. Cultures were washed three times in PBST, then incubated with donkey anti-sheep Alexa-488 (1:200, Invitrogen) together with phalloidin-Alexa-594 conjugate (20uM), in the blocking buffer for 45 minutes at room temperature. Cells were washed three times in PBST, then counterstained using 300nM 4',6-diamidino-2-phenylindole (DAPI) in PBS for 5 minutes at room temperature in the dark. Cultures were washed three times with PBST, twice with ddH₂O, and then mounted onto slides using Immu-mount (Thermo Fisher). Fluorescence was visualized with an Axioskop 2 microscope equipped with the appropriate fluorescence filters. Images were merged using Adobe Photoshop 7.0 (Figure 17).

### EXAMPLE 15

### APOPTOSIS TUNEL ASSAY

NSC34 cells were plated on German glass, photo-etched Coverslips (Electron Microscopy sciences) in 6-well plates at 200,000/well and cultured in 4ml of RPMI (with glutamine) for six days. At time of fixation, cells were washed twice in PBS, then fixed using 4% PFA/PBS for 20 minutes. After being rinsed three times in PBST, cells were incubated in permeabilization buffer (0.2% Triton X-100 in PBST) for 20 minutes. Cells were subsequently post-fixed for 10 minutes with 4% PFA/PBS. After being washed extensively with PBST, cells were stored at 4°C in sterile PBS.

At time of processing, cells were rinsed once with PBS, then overlaid with reaction solution from the Fluorescein *In Situ* Death Detection Kit (Roche Applied Science), as directed by manufacturer's instructions. Cells were incubated at 37°C for 1 hour, and then rinsed twice with PBST at room temperature in the dark. After rinsing three times in PBST, cells were counterstained with 300nm DAPI for 5 minutes in the dark. Cells were then rinsed twice with PBST, once with ddH₂O and then mounted onto slides using Immu-mount (Thermo Fisher). Fluorescence was visualized with an Axioskop2 microscope equipped with appropriate filters and total cells (DAPI) versus apoptotic cells (FITC) were counted manually by visual inspection (Figure 14, Panel C).

### EXAMPLE 16

### BROMODEOXYURIDINE (BRDU) PROLIFERATION ASSAY

NSC34 cells were plated on German glass, photo-etched Coverslips (Electron Microscopy Sciences) in 6-well plates at 200,000/well and cultured in 4ml of RPMI (with glutamine) for six days. 12 hours prior to fixation/processing, BrdU labeling solution was added to each well at a concentration of 10uM (Roche Applied Sciences). At the time of fixation, cells were washed three times in PBS to remove excess unincorporated BrdU, then fixed using 4% PFA/PBS for 20 minutes. After being rinsed three times in PBST, cells were incubated in permeabilization buffer (0.2% Triton X-100 in PBST) for 20 minutes. Cells were subsequently post-fixed for 10 minutes with 4% PFA/PBS. After being rinsed three times with PBST, the cells were placed in 0.1M sodium borate pH 8.5 for 2 minutes at room temperature.

The cultures were incubated in PBST with 0.5% (w/v) membrane blocking reagent (GE Healthcare) for one hour followed by the addition of anti-BrdU Alexa-488 (1:200, Invitrogen) for 45 minutes in blocking buffer at room temperature After rinsing three times in PBST, cells were counterstained with 300nm DAPI for 5 minutes in the dark. Cells were then rinsed twice with PBST, once with ddH₂O and then mounted onto slides using Immu-mount (Thermo Fisher). Fluorescence was visualized with an Axioskop2 microscope equipped with appropriate filters and total cells (DAPI) versus proliferating cells (Alexa-488) were counted manually by visual inspection (Figure 14, Panel B).

### EXAMPLE 17

### PRIMARY MOUSE MOTOR NEURONS

Dissociated primary motor neuron cultures were prepared from embryonic day 13 (E13) mice, plated on either 25mm or 14mm coverslips (Electron Microscopy Sciences), and grown for 4 to 7 weeks after dissociation [see Roy et al., J. Neurosci. 18:9673-9684 (1998)]. Cultures were fixed within the original plates using 4% PFA, rinsed twice with PBST, and incubated with permeabilization buffer (PBST with 0.2% Triton X-100) for 20 minutes. After being washed three times with PBST, the cultures were post-fixed for 10 minutes with 4% PFA, followed by extensive washing. Fixed cultures were incubated in PBST with 0.5% (w/v) membrane blocking reagent (GE Healthcare) along with 50ug/ml goat anti-mouse Fab (Rockland Immunochemicals) for one hour followed by the addition of sheep anti-mouse progranulin, (1:500 dilution, R&D Systems) and mouse anti-SMI 32, (1:1000, Sternberger Monoclonals).

Incubation with the primary antibody continued overnight at 4°C. Cultures were washed three times in PBST, then incubated with the donkey anti-sheep Alexa 594 (1:200, Invitrogen) and goat anti-mouse Alexa-488 (Invitrogen) in blocking buffer for 45 minutes at room temperature. Cells were washed three times in PBST, then counterstained using 300nM 4',6-diamidino-2-phenylindole (DAPI) in PBS for 5 minutes at room temperature in the dark. Cultures were washed three times with PBST, twice with ddH₂O, and mounted onto slides using Immu-mount (Thermo Fisher). Fluorescence was visualized with a Zeiss Axioskop 2 microscope equipped with the appropriate fluorescence filters. Images were merged using Adobe Photoshop 7.0 (Figure 12). Identification of primary motor neurons within the heterogeneous culture was based upon SMI32 immunoreactivity and cell body size [see Roy et al., J. Neurosci. 18:9673-9684 (1998)].

### EXAMPLE 18

### MOUSE SPINAL CORD CRYOSECTIONS

OCT mounted cryosections of 8 week old CD-1 mice were stored at -80°C prior to immunofluorescence. Cryosections were thawed at room temperature and fixed with 4% PFA, rinsed twice with PBST, and incubated with permeabilization buffer (PBST with 0.2% Triton X-100) for 20 minutes. After being washed three times with PBST, the cultures were post-fixed for 10 minutes with 4% PFA, followed by extensive washing. Fixed sections were incubated in PBST with 0.5% (w/v) membrane blocking reagent (GE Healthcare) along with 50ug/ml goat anti-mouse Fab (Rockland Immunochemicals) for one hour followed by the addition of sheep anti-mouse progranulin, (1:500 dilution, R&D Systems) and mouse anti-SMI 32, (1:1000, Sternberger Monoclonals).

Incubation with the primary antibody continued overnight at 4°C. Cultures were washed three times in PBST, and incubated with donkey anti-sheep Alexa 594 (1:200, Invitrogen) and goat anti-mouse Alexa-488 (Invitrogen) in blocking buffer for 45 minutes at room temperature. Cells were washed three times in PBST, and counterstained using 300nM 4',6-diamidino-2-phenylindole (DAPI) in PBS for 5 minutes at room temperature in the dark. Cultures were washed three times with PBST, twice with ddH₂O, and mounted onto slides using Immu-mount (Thermo Fisher). Fluorescence was visualized with a Zeiss Axioskop 2 microscope equipped with the appropriate fluorescence filters. Images were merged using Adobe Photoshop 7.0 (Figure 13). Identification of motor neurons within the tissue section was based upon SMI32 immunoreactivity and cell body size [see Roy et al., J. Neurosci. 18:9673-9684 (1998)].

### EXAMPLE 19

### AXOTOMY

Control mice (C57b1/6) mice underwent a unilateral proximal axotomy of the L3-L5 spinal roots and were autopsied at either day 3 or 7 post-axotomy. Mice were 6 weeks of age at time of surgery, and following axotomy allowed to recover with food and water ad libitum. Mice were anesthetized and killed by cardiac perfusion. Paraffin sections were obtained through the spinal level of origin of the motor units, and the sections stained for Progranulin immunoreactivity using routine immunohistochemistry (1:200, R&D Systems, overnight at 4°C, antigen retrieval for 7 minutes in a boiling high pH TRIS-EDTA buffer; secondary antibody development using Vector biotinylated anti-sheep and Vectastain Elite ABC kit and DAB visualization) (Figure 18).

### EXAMPLE 20

### PROTECTION AGAINST MPTP TOXICITY IN PC12 CELLS

PC12 cells were grown on collagen-coated 96-well plates, in Dulbecco's minimal essential medium (DMEM) in the presence of 10% fetal calf serum (FCS) supplemented with glutamine, penicillin and streptomycin. One day after plating (at 60-70% confluence, ≈ 40,000 cells/well), the growth medium was replaced by low-serum (2% FCS) medium with 4nM progranulin (PGRN+) or without progranulin (PGRN-) (Figure 19). Twenty-four hours later, the medium was removed and cells exposed to: 0, 100, 200, 500 or 1000 µM MPTP in the presence of DMEM containing 1% FCS. Following another 24 h of culture, the MPTP containing medium was removed and the methyl thiazolyl tetrazolium (MTT) colorimetric assay was performed to assess cell viability [see Zheng et al., In Vitro Cell Dev Biol Anim. 43(5-6):155-158 (2007)]

### EXAMPLE 21

### TREATMENT OF ALZHEIMER'S DISEASE

The Tg2576 mouse model of Alzheimer's Disease expresses the Swedish mutation of APP (APP_{K67ON,M671L}) at high levels under the control of the hamster prion protein promoter. These mice generate high levels of brain Aβ, and develop a progressive, age-related deposition in the form of amyloid plaques in the hippocampus, similar to that seen in humans. To assess the influence of progranulin on the development of these plaques, 8 month old mice were treated, via unilateral intrahippocampal infusion, with a recombinant lentiviral vector encoding either green fluorescent protein (GFP) or progranulin (PGRN). Animals were then sacrificed by perfusion at 12 months of age. Immunocytochemical analysis of Aβ deposition was performed on free-floating, 20 µm, coronal sections. For quantitative assessment, the total area occupied by anti-Aβ immunoreactive deposits was measured across 3 sections through the hippocampus. Figure 23 shows the survival of Tg2576 mice following intracerebral delivery of either GFP or PGRN expressing lentiviruses. Gene therapy with PGRN lentivirus results in increased survival of the amyloid transgenic mice.
*Animals*: Studies used 20-25 g female Tg2576 mice. Animals were housed in a temperature-controlled environment with a 12 h light/dark cycle and *ad libitum* access to standard chow and water. Procedures used in this study were approved by the Mayo Foundation Institutional Animal Care and Use Committee (IACUC).
*Viral vector delivery*: Animals were anaesthetized using isoflurane (1%) and placed in a Kopf stereotaxic frame. For hippocampal transduction, either GFP or PGRN lentiviral vector was injected unilaterally into the left hippocampus (A.P. -1.7, M.L. -1.5, D.V. -2.3) (2 µl/site) at a rate of 0.25 µl/minute via an infusion cannula connected by polyethylene tubing (50 PE) to a 50 µl Hamilton syringe driven by a Harvard pump. Following infusion, the vector was permitted to diffuse away from the cannula for four minutes before withdrawal.
*lmmunohistochemistry*: Mice were sacrificed by transcardial perfusion of 0.9% saline, the brains removed and post-fixed in 4% paraformaldehyde for immunohistochemical analysis. Symmetrical 20 µm-thick coronal sections were cut on a cryostat and stored in a Millonigs solution. Free-floating sections were pretreated with 70% formamide in Triton X-100/Tris-buffered saline [TBSt] at 37°C for 30 minutes and rinsed in TBSt. Sections were then incubated in 1% H₂O₂ in TBSt for 30 minutes, rinsed in TBSt, and incubated in blocking solution (5% goat serum/100mM lysine/0.3% TBSt) for 1 hour at room temperature, followed by incubation with the Aβ primary antibody (MM-27 33.1.1; 1:2000) overnight at room temperature. Sections were then incubated in a biotinylated secondary antibody followed by avidin-biotin-peroxidase complex using the Vectastain Elite kit. Sections were mounted on gelatin-coated slides and coverslipped with Entallen (Figure 20, Panel A).
*Quantitative analysis*: Surveys of Aβ deposition were performed in a 100X field in sections taken from the dorsal hippocampus. For quantitative assessment, the total area occupied by anti-Aβ immunoreactive deposits was measured in three anterior-posterior levels. Amyloid burden was calculated as the total area in the measurement field occupied by reaction product. Measurements were calculated for the entire hippocampal region contained within each section (Figure 20, Panel B). Unbiased stereological measurements were obtained using a computer-assisted image analysis system and Zeiss Axiovision 4.7 image analysis software. The investigator was blinded to treatment condition.
*Statisitical analysis*: Data were analyzed using an analysis of variance. Where significant F-values were obtained, planned pair-wise comparisons were made using Newman-Keuls. Differences were considered statistically significant when *p* < 0.05.

### EXAMPLE 22

### TREATMENT OF PARKINSON'S DISEASE

The influence of progranulin on dopaminergic neuronal cell loss in a 1-methyl-4-phenyl-1,2,3,6-tetrahyropyridine (MPTP)-induced mouse model of Parkinson's Disease, was determined by treatment of C57/BL6 mice, via unilateral intranigral infusion, with a lentiviral vector encoding either green fluorescent protein (GFP) or progranulin (PGRN). Three weeks later, animals received daily injections of MPTP (20 mg/kg, i.p.) for 5 days and were then sacrificed by perfusion 10 days following the last injection. Immunolabeling for TH was performed on free-floating, 20 µm, coronal sections. For quantitative assessment, the total number of TH⁺ cells was counted across 3 sections through the SNc.
*Animals*: Studies used 20-25 g male C57/B16 mice. Animals were housed in a temperature-controlled environment with a 12 h light/dark cycle and *ad libitum* access to standard chow and water. Procedures used in this study were approved by the institutional animal care committee.
*Viral vector delivery*: Animals were anaesthetized using isoflurane (1%) and placed in a Kopf stereotaxic frame. For intranigral transduction, either GFP or PGRN LV vector was injected unilaterally into the left SNc (A.P. -2.8, M.L. -1.3, D.V. -4.5) (2 µl/site) at a rate of 0.25 µl/minute via an infusion cannula connected by polyethylene tubing (50 PE) to a 50 µl Hamilton syringe driven by a Harvard pump. Following infusion, the vector was permitted to diffuse away from the cannula for four minutes before withdrawal.
*Immunohistochemistry*: Mice were sacrificed by transcardial perfusion of 0.9% saline, the brains removed and post-fixed in 4% paraformaldehyde for immunohistochemical analysis. Symmetrical 20 µm-thick coronal sections were cut on a cryostat and stored in a Millonigs solution. Free-floating sections were pretreated with 70% formamide in Triton X-100/Tris-buffered saline [TBSt] at 37°C for 30 minutes and rinsed in TBSt. Sections were then incubated in 1% H₂O₂ in TBSt for 30 minutes, rinsed in TBSt, and incubated in blocking solution (5% goat serum/100mM lysine/0.3% TBSt) for 1 hour at room temperature, followed by incubation with the Aβ primary antibody (MM-27 33.1.1; 1:2000) overnight at room temperature. Sections were then incubated in a biotinylated secondary antibody followed by avidin-biotin-peroxidase complex using the Vectastain Elite kit. Sections were mounted on gelatin-coated slides and coverslipped with Entallen (Figure 21, Panel A).
*Cell counting*: The number of immunopositive cell bodies was counted by an observer blinded to treatment history. Unbiased stereological cell counts were obtained using a computer-assisted image analysis system and Zeiss Axiovision 4.7 image analysis software. For counting cells of the substantia nigra, the compacta regions were defined by the distribution of TH-positivc cells within a set of clear anatomical landmarks/boundaries, used to delineate the SN_{c}. Immunopositive cells were counted using a 20X objective (sampling frame area, 90,000 µm²) containing an optical grid. The counting frame was placed over the counting area and then systematically moved in the X-Y direction until the entire delineated area was sampled. The number of immunopositive cells counted across 4 sections per animal was totaled for each animal (Figure 21, Panel B).
*Statisitical analysis*: Data were analyzed using a Student t-test, two-tailed with separate variance and a confidence interval of 95%. Differences were considered statistically significant when *p* < 0.05.

### EXAMPLE 23

### INCUBATION OF AN IMMORTALIZED MOTOR NEURON CELL LINE WITH GRANULIN EPITHELIN MODULES (GEMS) INFLUENCES CELL PROLIFERATION/SURVIVAL

NSC 34 cells (5000 cell/well) were plated in 96 well plates using 100ul of DMEM/10% FBS. The following day, the medium was removed and replaced with 100ul of RPMI (without serum) containing: 0, 50 or 100ng /ml of grn D or grn F. Cultures were incubated for 13 days following which cell proliferation/survival was determined using the CyQUANT® NF (kit #C35006) assay.

The CyQUANT® NF assay is based on measurement of cellular DNA content via fluorescent dye binding. The extent of proliferation/survival is determined by comparing fluorescence intensity for NSC-34 cells treated with GEMs (50 and 100ng) relative to untreated controls (0ng).

As per the manufacturers instructions, the protocol included aspiration of growth medium, replacement with 100ul of dye binding solution per well, incubation for 30 minutes following which the fluorescence intensity of each sample was measured using a fluorescence microplate reader with excitation at ∼485 nm and emission detection at ∼530 nm (Figure 22). The immortalized motor neuron cell line (NSC-34) responded to incubation with grn F and grn D with either proliferation/survival (grn F) or no effect (grn D) similar to the response of extra neuronal cells incubated in the presence of these GEM's (described in: Tolkatchev D. et al., Protein Sci. 17:711-724, 2008).

### EXAMPLE 24

### PROGRANULIN EXPRESSING LENTIVIRUS PROTECTS BSSG CHALLENGED MICE

Figure 24 shows spinal motor neuron counts and choline acetyl transferase activity in PGRN lentivirus treated mice. Motor neuron counts were assessed by Nissl stain. Motor neuron counts were more normal in BSSG exposed PGRN-lentivirus treated mice relative to control (saline treated) BSSG exposed mice, with a P value (Student t-test) approaching significance at 0.068. Immunohistolochemical assessment of choline acetyl transferase (ChAT) suggested decreased activity of this motor neuron marker in anterior horn cells of saline treated BSSG exposed mice relative to all other treatment groups (upper right hand panels of Figure 24, Panel (B). For Nissl staining, the numbers of ChAT positive motor neurons (Figure 24, Panel C) appeared to be reduced in control (saline treated) BSSG exposed mice.
*Lentiviral vector*: The progranulin-expressing lentiviral vector was designed and produced under contract by Invitrogen Corporation (Carlsbad, CA). The titer was determined to be 1X 10⁸ TU/mL by the blasticidin resistance assay. The lentivirus was stored in cryovials frozen at -80°C until the day of injection.
*Animals*: Male CD-1 mice obtained from Charles River Laboratories (Wilmington, MA) were singly housed at 22°C on a 12:12 h light-dark cycle. Forty animals were randomly divided into 4 groups: i) PGRN-LV injected with BSSG feeding, ii) PGRN-LV injected with normal mouse chow, iii) saline-injected with BSSG feeding, and iv) saline-injected with normal mouse chow. Experimental manipulations were approved by the University of British Columbia Committee on Animal Care.
*Viral Administration*: At three months of age, male CD-1 mice receiving the progranulin-expressing lentivirus and saline injected control mice, were anesthetized using isoflurane and the lentiviral vector or saline control delivered via direct injection into the right gastrocnemius muscle. Five injections each consisting of 5µl (1x10⁸ TU/ml) were performed in order to increase the number of motor neurons transduced.
*BSSG Administration*: β-Sitosterol β-D-glucoside (BSSG) was synthesized under contract basis to the Department of Chemistry at the University of British Columbia. The synthesized compound was characterized using NMR (¹H and ¹³C) and high-resolution mass spectrometry. A purity of at least 95% was verified by HPLC. To create the experimental pellet at the desired concentration (2 mg of BSSG/day), BSSG was mixed with ground up mouse pellets (Mouse Diet, Lab Diet®, Richmond, IN). The feeding paradigm was initialized three weeks following the intramuscular injections. Treated pellets were provided each morning with access to regular chow restored ad libitum in the afternoon once the animals had ingested the provided pellet. In general, all of the mice in the BSSG group routinely ate the entire pellet. Control mice were fed only normal mouse chow. BSSG feeding was conducted daily for 15 weeks followed by a 5 week washout period.
*Histology*: At the time of sacrifice, the animals were anaesthetized with halothane and perfused via a cardiac puncture with chilled PBS and 4% paraformaldehyde (PFA). Spinal cord and brain samples were removed and immersed in 4% PFA for 2 days, cryoprotected in 30% sucrose in 0.01M phosphate-buffered saline (PBS) solution, pH 7.4 for 1 day, and then stored frozen at -80°C until sectioning for immunohistology on a Leica CM3050 S (Leica Microsystems, Nussloch, Germany) motorized cryostat. Spinal cords were serially sectioned at 20 µm. Lumbar spinal cord (L4-L6) was sectioned in the coronal plane [Wilson et al., Neuromolecular Medicine, 3, 105-118 (2003); Wilson et al., Neuroimage, 23, 336-343 (2004)]. Immunohistochemistry was performed at the same time for sections for all animals. Microscopy of the stained sections and recording of the level of labeling was conducted by observers blinded to the identity of the mice.
*Nissl Stain*: Motor neuron counts were determined using the Nissl stain. A solution of 0.5% cresyl violet was made by adding 0.5g of cresyl violet acetate (Sigma-Aldrich Inc., St. Louis, MO) to 100mL of warm ddH₂O, then acidifying with 10 drops of glacial acetic acid. After the solution has been mixed and cooled, it was filtered through filter paper (Whatman® #1). Slide-mounted sections were first rinsed twice in 1XPBS for 2 minutes to remove excess O.C.T. Then the sections were placed in 95% ethanol (5 minutes), 70% ethanol (3 minutes) and dH₂O (2 minutes). The slides were left to stain in 0.5% cresyl violet solution for 3 minutes. After staining, the slides were rinsed in ddH₂O for 1 minute, then dehydrated in 70% ethanol + 1% acetic acid (1.5 minutes), 70% ethanol (30 seconds), 95% ethanol (2 minutes), two changes of 100% ethanol (several dips) and two changes of xylene (several dips). The slides were allowed to dry before mounting in Clarion™ mounting medium (Sigma-Aldrich Inc., St. Louis, MO).
*Immunohistochemistry*: Choline acetyl transferase (ChAT, Millipore, Billerica, MA), levels were determined using the non-fluorescent diaminobenzidine method. Slides containing mounted sections of lumbar spinal cord were first rinsed twice in 1X PBS (5 minutes). Endogenous peroxidase activity was quenched using 3% hydrogen peroxide in PBST (PBS + 0.5% Triton X-100) for 5 minutes. The sections were rinsed twice in 1X PBS (2 minutes) before blocking at room temperature (RT) for 1 hour in 10% normal serum + 1% bovine serum albumin (BSA) in PBST. The primary antibody was diluted in 1% normal serum + 1% BSA in PBST. Dilutions and incubation time and temperature were as follows: ChAT (1:100 for 1hour at RT). After the primary antibody incubation step, the slides were rinsed twice in 1X PBS, and then incubated in the secondary antibody (Vectastain ABC Elite Kit, Vector Laboratories Inc., Burlingame, CA) for 1 hour at RT. The sections were rinsed in 1X PBS (2X 2 minutes) before incubating in the Vectorstain ABC Elite Reagents for another 30 minutes at RT. The slides were rinsed again in 1X PBS (2X 2 minutes). Color development was done using the Vector peroxidase substrate kit - DAB. It took 1-2 minutes for the desirable brown color to develop. When the desirable color was achieved, the slides were rinsed in ddH₂O for 5 minutes and counter-stained in 0.5% methyl green for ten minutes. After counter-staining, the slides were rinsed briefly in dH₂O, two changes of 95% ethanol and two changes of 100% ethanol. Slides were allowed to dry before they were mounted in mounting medium.
*Microscopy*: Sections were visualized using the Motic B5 Professional Series 3.0 microscope (Motic Instruments Inc., Richmond, ON) and images were captured using the Motic Images Advanced 3.0 software.

### EXAMPLE 25

### PROGRANULIN OVER EXPRESSION PROTECTS NSC-34 CELLS FROM BSSG-INDUCED TOXICITY

Normal NSC-34 and a stable NSC-34 transfectant that over expressed human PGRN (NSC-34-hPGRN) NCS34 were maintained in culture using DMEM supplemented with 5% (v/v) fetal calf serum (FCS). Cells were aliquoted in 96-well plates at a density of 8,000cells/well in 200uL of DMEM 5%FCS.d After two hours the neurotoxin Beta-sitosteryl glucoside (BSSG) was added at concentrations of 0, 100, 250, 500, 1000 and 2500ng/mL with a final culture volume of 250uL along with 0.1% (v/v) DMSO throughout. As a negative control for cell proliferation/survival, a series of wells were washed and media replaced with DMEM containing no FCS. Following 72 hours cell viability was measured using the MTT assay (Vybrant MTT Cell proliferation Assay; Invitrogen) according to manufacturer's instructions.

NSC-34 cells are an immortalized model of motor neuron function and BSSG has been shown to cause cytotoxicity in this cell line [Cashman et al., Dev Dyn 1992, 194(3):209-221; Tabata et al., Neuromolecular Med 2008, 10(1):24-39]. Progranulin (PGRN) promotes growth and survival in numerous cell lines including an increased survival of rat motor neurons under serum deprivation [Van Dammeet al., J Cell Biol 2008, 181(1):37-41]. To determine if PGRN was capable of protecting NSC-34 cells against BSSG neurotoxicity a stable transfectant was developed to over express human PGRN constitutively. Progranulin protected NSC-34 cells during a 72 hour incubation with 1000 and 2500ng/mL of BSSG (Figure 25). In addition, PGRN over expression increased cell survival in serum free cultures.

### EXAMPLE 26

### PROGRANULIN PROTEIN IS CAPABLE OF PROTECTING THE IMMMORTALIZED MOTOR NEURON CELL LINE NSC-34 FROM SERUM DEPRIVATION

Human recombinant progranulin (PGRN) protein added to NSC-34 cells in culture resulted in a 2.5 fold (Day 4) increase cell survival following serum starvation (Figure 26). Normal NSC-34 cells were maintained in culture using DMEM supplemented with 5% (v/v) fetal calf serum (FCS). Cells were aliquoted in 96-well plates at a density of 6,000cells/well in 200uL of DMEM 5%FCS. After 2 hours of culture (to ensure cell adhesion) media was removed, cells washed 1x with PBS and DMEM serum free medium with or without hPGRN (100 ng/ml) added to the cells. Following one and four days of culture remaining metabolically active cells were detected using the Alamar Blue® methodology (Molecular Probes/Invitrogen) following the manufacturer's instructions. This data indicates the conservation of function of PGRN proteins.

### EXAMPLE 27

### IN VIVO DEMONSTRATION OF PARTIAL PROGRANULIN RESCUE OF A DEVELOPMENT/SURVIVAL DEFECT IN THE ZEBRAFISH MOTOR SYSTEM

During the first day of zebrafish development, neuromuscular connections are restricted to the three primary motoneurons (CaP, MiP and RoP) per spinal cord hemisegment where they innervate three myotome areas that ultimately develop into body wall muscle. During the first the 24hpf CaP axons project to establish the common pathway. This is the developmental period encompassed in Figures 27 and 28.

Development of ventral primary neurons were examined in whole mount embryos at 26-28hpf and visualized by immunostaining with znp1 monoclonal antibody that labels primary motor neurons. (Wild type development of Cap neurons exhibits branching only beyond the "choice point" (Figure 27, Panel F, double arrowheads).

Knockdown of progranulin-A (using an MO directed towards the 5'UTR) generated a range of morphant phenotypes ranging from truncation (Figure 27, Panel A, B, black arrows), premature branching (Figure 27, Panel B, black arrowheads) to complete absence of primary motorneurons (Figure 27, Panel C). Co-injection of progranulin mRNA with progranulin-A MO produces a partial rescue (Figure 27,, Panels D and E). There is reduced incidence (P<0.001) of truncated neurons (Figure 27, Panel D, white arrows) together with increased incidence (P<0.001) of early and late branching (Figure 27, Panel E, white arrowheads) as shown in Table 2. Data sets were accumulated from 50 embryos (WT, progranulin MO, progranulin MO plus mRNA). These data suggest that development of branched ventral motorneurons is very sensitive to progranulin-A over or under expression.

**Table 2. Progranulin knockdown induces aberrant ventral motor axon/nerve growth**

| **Injection type** | **Truncation** | **Branching** |
|---|---|---|
| Wt (no injection) | 0.16±0.07 | 0.32±0.12 |
| PGRN-MO2 | 3.02±0.31***a | 1.0±0.19*c |
| PGRN-MO2 + 100 pg (PGRN) | **0.94±0.19*****b | 2.8±0.32***d |

| | | |
|---|---|---|
| Average number of specified motor axon/nerve defects per affected side (n=50 each group) Significance was determined by student-Newman-keuls Multiple Comparisons Test a- comparison of WT truncation vs MO2 p<0.001 b- comparison of MO2 truncation vs MO2+100pgpgrnA mRNA p<0.001 c- comparison of WT branching vs MO2 p<0.05 d- comparison of MO2 branching vs MO2 +100pgpgrnA mRNA p<0.001 | | |

Smn1 knockdown resulted in truncated (Figure 28, Panel A; black arrows) and branched motor axons/nerves (Figure 28, Panel B; black arrowheads). Although the mean value of truncation between Smn1 MO versus co-injection of Smn1 MO with progranulin-A mRNA is not statistically significant, the reduced mean value from 0.38 to 0.18 per affected side of embryo confirms partial rescue of the truncation defect (Table: 3, Figure 28, Panel C; white arrow) and increases branched axons/nerves similar to the results shown in Figure 27 (Panel C; white arrowheads) and Table 3.

**Table 3. Co-injection of Smn1 MO and 100pg of ProgranulinA mRNA reduces truncation and increases branching of ventral motor axon/ nerve growth**

| **Injection Type** | **Truncation** | **Branching** |
|---|---|---|
| WT | 0.10±0.04 | 0.38±0.08 |
| Smn1MO | 0.38±0.01 | 1.52±0.23ₐ |
| Smn1MO + 100pg PgrnA mRNA | **0.18±0.08** | 2.22±0.31_{b} |
| 100pg PgrnA mRNA | 0.10±0.04 | 1.36±0.22_{c} |

| | | |
|---|---|---|
| Significance was determined by student-Newman-keuls Multiple Comparisons Test a- Comparison of WT branching vs Smn1MO p<0.001 b- Comparison of WT branching vs Smn1MO+ 100pg progranulinA mRNA p<0.001 c- Comparison of WT branching vs 100pg progranulinA mRNA p<0.001 | | |

*Fish husbandry*: Wild type zebrafish were purchased from Aquatica Tropicals (Florida) and maintained on a 14h/10h light/dark cycle at 28.5°C in a laboratory aquarium (Allentown Caging Equipment Co. Inc., Allentown, NJ). Fish were fed twice daily. In the late afternoon of the day before eggs are required, fish were transferred to a net positioned towards the top of a holding tank and covered. In the morning, after the light cycle begins and spawning has stopped, the eggs that have fallen through the net were collected from the bottom of the tank. Embryos to be used for developmental studies were collected and staged by hours post fertilization (hpf).
*Embryo microinjection of morpholino oligonucleotides*: Morpholino oligonucleotides (MO) were obtained from Gene Tools, Inc. (Philomath, OR) and diluted in Danieaux buffer (58 mM NaCl, 0.7 mM KCl, 0.4 mM MgSO4, 0.6 mM Ca(NO3)₂, 5.0 mM Hepes pH 7.6) containing 0.1% phenol red (Nasevicius and Ekker 2000). Approximately 2 nL of Morpholino along with 0.05% FITC-dextran (Sigma-Aldrich, Oakville, ON, Canada) was injected into the yolk of 1- to 2-cell stage embryos using a PLI-100 microinjection system (Harvard Apparatus, St. Laurent, QC, Canada). Phenotype observation and documentation were accomplished using a Leica DC300F digital camera connected to a Leica MZFLIII stereomicroscope and processed with Adobe Photoshop 7.0 software. The sequence of the 5'UTR region of PgrnA morpholino (MO) was 5'GAGCAGGTGGATTTGTGAA CAGCGG3'. Morpholinos against the initiator AUG of Smn1 (5'CGACATCTTCTGCACCATTGGC3'), and Smn1 UTR (5'TTTAAATATTTCCCAAGTCCAACGT) were also used. For Morpholino injection 10ng PgrnA, or 9ng of Smn1 were used.
*Microinjection of I'grn mRNA*: For Pgrn mRNA over-expression and rescue experiments the vector was generated as follows: The full-length progranulin-a sequence was purchased from RZPD (Berlin, Germany) as clone UCDMp574E2318Q2 and subcloned into pcDNA3.1-V5/His vector (Invitrogen, Carlsbad, CA) using a forward primer that overlapped with the starter AUG a reverse primer that read through the termination codon. The final vector constructs consisted of full-length progranulin-A with a carboxyl-terminal tag consisting of the V5 epitope and 6xHistidine. The morpholino was designed against the 5'UTR region. Since the construct for mRNA microinjection does not contain untranslated 5' sequence there is no possibility of binding between mRNA and the morpholino when they are co-injected. Translation enhanced capped mRNA was synthesized with the mMessage mMachine Kit (Ambion, Huntingdon, England). For mRNA overexpression or rescue experiments 100pg of Pgrn A mRNA was used.

Microinjection of a vector encoding GFP was used as a control to demonstrate that the microinjection and over-expression does not inherently affect development. Green Fluorescent Protein cloned in the pcDNA3 vector was first transcribed and injected up to 1ng per embryo and signal was observed under enhanced GFP filter using Leica MZFLIII stereomicroscope. The fluorescent GFP signal confirms that the mRNA was intact and translated into to protein.
*Immunohistochemistry*: Embryos (approximately 26-28 hpf) grown in Danieaux buffer were supplemented with 0.003% of the tyrosinase inhibitor 1-phenyl-2-thiourea (phenythiocarbamide P-5272, Sigma-Aldrich) to prevent the appearance of melanin pigmentation. Staged embryos were manually dechorionated and fixed for 2 hours at room temperature or overnight at 4°C in 4% parafonnaldehyde/PBS. After several washes in PBS, embryos were stored in 100% methanol until required. Rehydration of embryos was performed for 5 min each in successive solutions of MeOH / PBST and then 3 times in PBST. Embryos were permeabilized with proteinase K diluted in PBST at a final concentration of 10 µg/ml. Post fixation was then carried out in 4% PFA/PBS for 20 min at room temperature followed by 3 rinses in PBST. Embryos were incubated with blocking buffer (5% Calf Serum, 1% DMSO in PBST for 3-5 hours. Embryos were incubated with znp1 (ZIRC) monoclonal antibody (1:200) which was diluted in blocking buffer. Incubations were carried overnight at 4°C followed by six washes in PBST. Embryos were then incubated with Goat anti-Mouse AP conjugate (Calbiochem) secondary antibody diluted to 1:200 with blocking buffer in PBST for 2hr at room temperature followed by six washes in PBST. Embryos were incubated in staining buffer (100mM Tris-Hcl pH 9.5, 50mM MgCl₂, 100mM NaCl, 0.1% Tween-20,1mM levamisol) with NBT and BCIP-T. After 30min staining was stopped and Caudal primary motor neurons (PMN) within the trunk (excluding the tail region) of the embryos were visualized under a Olympus inverted phase contrast microscope. Pictures were taken with a Olympus DP12 camera and processed with Adobe Photoshop 7.0 software.
*Analysis of PMN subtype growth*: Caudal primary motor axons in whole-mounted 26-28hpf embryos labelled with Znp1 monoclonal antibody were analysed. Only the trunk PMNs (12 pairs) were scored. All of these had grown beyond the ventral edge of the notochord into the ventral somite in wild type embryos at 24hpf. Trunk hemisegments were scored as 'branched' when nerves were branched at or above the ventral edge of the notochord. This strategy was employed to exclude naturally occurring branching that is sometimes observed ventral to the notochord. Trunk hemisegments were also scored as 'truncated' when nerves did not grow beyond the horizontal myoseptum. When more than one znp1 immunolabeled axon fascicle exited the spinal cord this was scored as a multiple exit. Embryos with respective phenotypes were numbered and expressed as a percentage. Embryos were also counted with respect to how many of the nerves of the 12 pairs in each demonstrated a particular defect. For each treatment at least three experiments were performed. Values were expressed as mean ± standard error of the mean. Statistical analysis were done using student-Newman-keuls Multiple Comparisons Test.

## Claims

1. A composition comprising an effector that increases progranulin expression or a progranulin polypeptide for use in treating a neurodegenerative disease in a patient, wherein the effector is a nucleic acid encoding progranulin.

2. The composition for use according to claim 1, wherein the composition is adapted for parenteral administration, and wherein the route of parenteral administration is selected from the group consisting of intradermally, subcutaneously, intramuscularly, intraperitoneally, intravenously, intraventricularly, intrathecally, intracerebrally, and intracordally.

3. The composition for use according to any one of claims 1 to 2, wherein the neurodegenerative disease is not caused by a heritable mutation of the progranulin gene that modifies progranulin expression.

4. The composition for use according to any one of claims 1 to 3, wherein the neurodegenerative disease is mediated by an excitotoxin, and wherein the excitotoxin is a sterol glycoside.

5. The composition for use according to claim 4, wherein the sterol glycoside is selected from the group consisting of beta-sitosterol-beta-D-glucoside, stigmasterol glucoside, and cholestrol glucoside, or analogs or derivatives thereof.

6. The composition for use according to any one of claims 1 to 5, wherein the neurodegenerative disease is selected from the group consisting of motor neuron disease and amyotrophic lateral sclerosis.

7. The composition for use according to any one of claims 1 to 6, wherein the neurodegenerative disease is motor neuron disease.

8. The composition for use according to any one of claims 1 to 6, wherein the neurodegenerative disease is amyotrophic lateral sclerosis.

9. A pharmaceutical composition comprising an effector that increases progranulin expression or a progranulin polypeptide and a pharmaceutically acceptable carrier therefor for use in reducing or preventing the symptoms of a neurodegenerative disease in a patient, wherein the effector is a nucleic acid encoding progranulin.

10. The pharmaceutical composition for use according to claim 9, wherein the composition is capable of increasing progranulin expression in neurons.

11. The pharmaceutical composition for use according to any one of claims 9 to 10 in a parenteral dosage form, wherein the dosage form is adapted for parenteral administration by a route selected from the group consisting of intradermal, subcutaneous, intramuscular, intraperitoneal, intravenous, intraventicular, intrathecal, intracerebral and intracordal.

12. The pharmaceutical composition for use according to any one of claims 9 to 11, wherein the neurodegenerative disease is not caused by a heritable mutation of the progranulin gene that modifies progranulin expression.

13. The pharmaceutical composition for use according to any one of claims 9 to 12, wherein the neurodegenerative disease is mediated by an excitotoxin, wherein the excitotoxin is a sterol glycoside, and wherein the sterol glycoside is selected from the group consisting of beta-sitosterol-beta-D-glucoside, stigmasterol glucoside and cholesterol glucoside, or analogs or derivatives thereof.

14. The pharmaceutical composition for use according to any one of claims 9 to 13, wherein the neurodegenerative disease is motor neuron disease.

15. The pharmaceutical composition for use according to any one of claims 9 to 13, wherein the neurodegenerative disease is amyotrophic lateral sclerosis.

## Patentansprüche

1. Zusammensetzung, umfassend einen Effektor, der die Progranulinexpression erhöht, oder ein Progranulin-Polypeptid zur Verwendung bei der Behandlung einer neurodegenerativen Erkrankung in einem Patienten, wobei der Effektor eine Progranulin kodierende Nukleinsäure ist.

2. Die Zusammensetzung zur Verwendung nach Anspruch 1, wobei die Zusammensetzung zur parenteralen Verabreichung angepasst ist, und wobei der Weg der parenteralen Verabreichung ausgewählt ist aus der Gruppe bestehend aus intradermal, subkutan, intramuskulär, intraperitoneal, intravenös, intraventrikulär, intrathekal, intrazerebral und intracordal.

3. Die Zusammensetzung zur Verwendung nach einem beliebigen der Ansprüche 1 bis 2, wobei die neurodegenerative Erkrankung nicht durch eine vererbbare Mutation des Progranulin-Gens, welche die Progranulinexpression modifiziert, vermittelt wird.

4. Die Zusammensetzung zur Verwendung nach einem beliebigen der Ansprüche 1 bis 3, wobei die neurodegenerative Erkrankung durch ein Exzitotoxin vermittelt wird und wobei das Exzitotoxin ein Sterolglycosid ist.

5. Die Zusammensetzung zur Verwendung nach Anspruch 4, wobei das Sterolglycosid ausgewählt ist aus der Gruppe bestehend aus beta-Sitosterol-beta-D-Glucosid, Stigmasterolglucosid und Cholestrolglucosid oder Analoga oder Derivaten von diesen.

6. Die Zusammensetzung zur Verwendung nach einem beliebigen der Ansprüche 1 bis 5, wobei die neurodegenerative Erkrankung ausgewählt ist aus der Gruppe bestehend aus Motoneuronenerkrankung und amyotropher Lateralsklerose.

7. Die Zusammensetzung zur Verwendung nach einem beliebigen der Ansprüche 1 bis 6, wobei die neurodegenerative Erkrankung Motoneuronerkrankung ist.

8. Die Zusammensetzung zur Verwendung nach einem beliebigen der Ansprüche 1 bis 6, wobei die neurodegenerative Erkrankung amyotrophe Lateralsklerose ist.

9. Eine pharmazeutische Zusammensetzung, umfassend einen Effektor, der die ProgranulinExpression erhöht, oder ein Progranulin-Polypeptid und einen pharmazeutisch verträglichen Träger dafür, zur Verwendung in der Reduktion oder Prävention der Symptome einer neurodegenerativen Erkrankung bei einem Patienten, wobei der Effektor eine Progranulin kodierende Nukleinsäure ist.

10. Die pharmazeutische Zusammensetzung zur Verwendung nach Anspruch 9, wobei die Zusammensetzung in der Lage ist, die Progranulinexpression in Neuronen zu erhöhen.

11. Die pharmazeutische Zusammensetzung zur Verwendung nach einem beliebigen der Ansprüche 9 bis 10 in einer parenteralen Dosierungsform, wobei die Dosierungsform zur parenteralen Verabreichung auf einem Weg ausgewählt aus der Gruppe bestehend aus intradermal, subkutan, intramuskulär, intraperitoneal, intravenös, intraventrikulär, intrathekal, intrazerebral und intracordal angepasst ist.

12. Die pharmazeutische Zusammensetzung zur Verwendung nach einem beliebigen der Ansprüche 9 bis 11, wobei die neurodegenerative Erkrankung nicht durch eine vererbbare Mutation des Progranulin-Gens, welche die Progranulinexpression modifiziert, vermittelt wird.

13. Die pharmazeutische Zusammensetzung zur Verwendung nach einem beliebigen der Ansprüche 9 bis 12, wobei die neurodegenerative Erkrankung durch ein Exzitotoxin vermittelt wird, wobei das Exzitotoxin ein Sterolglycosid ist, und wobei das Sterolglycosid ausgewählt ist aus der Gruppe bestehend aus beta-Sitosterol-beta-D- Glucosid, Stigmasterolglucosid und Cholesterolglucosid oder Analoga oder Derivate von diesen.

14. Die pharmazeutische Zusammensetzung zur Verwendung nach einem beliebigen der Ansprüche 9 bis 13, wobei die neurodegenerative Erkrankung Motoneuronerkrankung ist.

15. Die pharmazeutische Zusammensetzung zur Verwendung nach einem beliebigen der Ansprüche 9 bis 13, wobei die neurodegenerative Erkrankung amyotrophe Lateralsklerose ist.

## Revendications

1. Composition comprenant un effecteur qui augmente l'expression de la progranuline ou d'un polypeptide de progranuline, pour une utilisation dans le traitement d'une maladie neurodégénérative chez un patient, où l'effecteur est un acide nucléique codant pour la progranuline.

2. Composition pour une utilisation selon la revendication 1, où la composition est adaptée pour une administration parentérale, et où la voie d'administration parentérale est choisie dans le groupe constitué par les voies intradermique, sous-cutanée, intramusculaire, intrapéritonéale, intraveineuse, intraventriculaire, intrathécale, intracérébrale et intracordale.

3. Composition pour une utilisation selon l'une quelconque des revendications 1 à 2, où la maladie neurodégénérative n'est pas provoquée par une mutation héréditaire du gène de progranuline qui modifie l'expression de la progranuline.

4. Composition pour une utilisation selon l'une quelconque des revendications 1 à 3, où la maladie neurodégénérative est médiée par une excitotoxine, et où l'excitotoxine est un glycoside de stérol.

5. Composition pour une utilisation selon la revendication 4, où le glycoside de stérol est choisi dans le groupe constitué par le bêta-sitostérol-bêta-D-glucoside, le glucoside de stigmastérol, et le glucoside de cholestérol, ou des analogues ou dérivés de ceux-ci.

6. Composition pour une utilisation selon l'une quelconque des revendications 1 à 5, où la maladie neurodégénérative est choisie dans le groupe constitué par la maladie des motoneurones et la sclérose latérale amyotrophique.

7. Composition pour une utilisation selon l'une quelconque des revendications 1 à 6, où la maladie neurodégénérative est la maladie des motoneurones.

8. Composition pour une utilisation selon l'une quelconque des revendications 1 à 6, où la maladie neurodégénérative est la sclérose latérale amyotrophique.

9. Composition pharmaceutique comprenant un effecteur qui augmente l'expression de la progranuline ou d'un polypeptide de progranuline et un véhicule pharmaceutiquement acceptable pour celui-ci, pour une utilisation dans la réduction ou la prévention des symptômes d'une maladie neurodégénérative chez un patient, où l'effecteur est un acide nucléique codant pour la progranuline.

10. Composition pharmaceutique pour une utilisation selon la revendication 9, où la composition est capable d'augmenter l'expression de la progranuline dans les neurones.

11. Composition pharmaceutique pour une utilisation selon l'une quelconque des revendications 9 à 10 sous une forme de dosage parentérale, où la forme de dosage est adaptée pour une administration parentérale par une voie choisie dans le groupe constitué par les voies intradermique, sous-cutanée, intramusculaire, intrapéritonéale, intraveineuse, intraventriculaire, intrathécale, intracérébrale et intracordale.

12. Composition pharmaceutique pour une utilisation selon l'une quelconque des revendications 9 à 11, où la maladie neurodégénérative n'est pas provoquée par une mutation héréditaire du gène de progranuline qui modifie l'expression de la progranuline.

13. Composition pharmaceutique pour une utilisation selon l'une quelconque des revendications 9 à 12, où la maladie neurodégénérative est médiée par une excitotoxine, où l'excitotoxine est un glycoside de stérol, et où le glycoside de stérol est choisi dans le groupe constitué par le bêta-sitostérol-bêta-D-glucoside, le glucoside de stigmastérol, et le glucoside de cholestérol, ou des analogues ou dérivés de ceux-ci.

14. Composition pharmaceutique pour une utilisation selon l'une quelconque des revendications 9 à 13, où la maladie neurodégénérative est la maladie des motoneurones.

15. Composition pharmaceutique pour une utilisation selon l'une quelconque des revendications 9 à 13, où la maladie neurodégénérative est la sclérose latérale amyotrophique.
